# EUROPEAN PATENT APPLICATION

(11) **EP 4 799 601 A1**
(43) Date of publication of application: **02.09.2026**
(21) Application number: 25160701.6
(22) Date of filing: 27.02.2025
(51) Int. Cl.: A61K 8/02, A61K 8/04, A61K 8/73, A61K 8/88, A61K 9/00, A61K 9/10, A61K 9/50, A61K 9/51, A61K 38/00, A61Q 11/00

(54) **ENCAPSULATED COMPOSITIONS FOR DENTAL AND BONE REMINERALISATION**

(71) Applicant: vVardis AG, 6300 Zug (CH)
(72) Inventor: PAPADEA, Konstantina., 6300 Zug (CH); SHAKHOVA, Olga., 6300 Zug (CH); CARTIER, Régis., 6300 Zug (CH); ABIVARDI BRÖNNER, Haleh., 6300 Zug (CH); ABIVARDI SIGNER, Golnar., 6300 Zug (CH)
(74) Representative: Moré, Solveig Helga

(57) **Abstract**

The present invention relates to the field of medicinal tissue mineralisation, in particular, i.e. tooth remineralisation and bone regeneration with self-assembling peptides. Use of self-assembling peptides, such as P11-4, also designated Oligopeptide-104, in these processes leads to generation of hydroxyapatite, which is also present in natural enamel, dentin and bone. The inventors have discovered that this can be significantly accelerated by combining a self-assembling peptide (SAP) such as P11-4, calcium ions, phosphate ions, and a polymer in the form of particles encapsulating a cargo selected from at least one of the SAP, the calcium ions and the phosphate ions into a composition or kit. The invention also provides medical use of said composition or kit, in particular, in the tooth, for remineralisation of caries lesions, such as subsurface caries lesions, mineralisation of pits and fissures, treatment of sensitive teeth, pulp capping, and for bone regeneration.

## Description

The present invention relates to the field of medicinal tissue mineralisation, in particular, tooth remineralisation and bone regeneration with self-assembling peptides. Use of self-assembling peptides, such as P11-4 (also designated Oligopeptide-104), in these processes leads to generation of hydroxyapatite, which is also present in natural enamel, dentin and bone. The inventors have discovered that this can be significantly accelerated by combining a self-assembling peptide (SAP) such as P11-4, calcium ions, phosphate ions, and a polymer in the form of particles encapsulating a cargo selected from at least one of the SAP, the calcium ions and the phosphate ions into a composition or kit. The invention also provides medical use of said composition or kit, in particular, for remineralisation of caries lesions, such as subsurface caries lesions, mineralisation of pits and fissures, treatment of sensitive teeth, pulp capping, and for bone regeneration.

Tooth decay, also known as dental caries, is one of the most ubiquitous diseases in the world caused by acid secreting bacteria. It is a breakdown of tooth material due to bacterial metabolites, mainly acids made by bacteria when they break down food debris or sugar on the surface or in the oral biofilm. This leads to an imbalance between demineralisation and remineralisation processes. Hard tooth structures, i.e., enamel, dentin and cementum, are damaged by ongoing demineralisation, which results in carious lesions and eventually in the appearance of dental cavities. The earliest sign of a new carious lesion is the appearance of a chalky white spot on the surface of the tooth, a so-called white spot lesion, or subsurface caries lesion (or subsurface lesion, also designated an incipient carious lesion). As the demineralisation progresses, the mineralised surface of the lesion (partially) collapses and breaks and a microcavity or a cavity, a hole in the tooth, appears. This is referred to as a (partially) cavitated carious lesion or cavity.

The classical treatment for tooth lesions, in particular, carious tooth lesions, is drilling and filling the tooth. Once a carious lesion has cavitated and a hole in the tooth appears, the common treatment applied to date is invasive. The decayed material is usually removed by using, for example, a dental handpiece ("drill"). Alternatively, a laser, a dental spoon or a chemo-mechanical system may be used to remove dental caries.

After removal (i.e. excavation) of the carious enamel and/or dentin, the missing tooth structure requires a dental restoration using dental restorative materials such as sealants, amalgam, dental composites, porcelain or gold.

However, it is advantageous to be able to restore the natural tooth matter, which is based on hydroxyapatite, instead of losing precious hard tissue followed by filling excavated lesions with a substance foreign to the human body. Such seamless remineralisation can, e.g., reduce the incidence of secondary caries that often appears at the filling margins, or when a filling fails.

Non-invasive approaches based on remineralisation have been suggested for treatment of non-cavitated carious lesions, i.e. subsurface lesions. For example, classically, remineralisation is attempted by the application of topical fluoride.

In WO 2017/168183 A1, a biomimetic mineralised apatite structure based on elastin-like peptides is described for use as dental restorative material, in particular for the reconstruction of enamel, and for use in the treatment of dental diseases such as dental caries.

Furthermore, casein-based strategies have been developed for remineralisation of enamel and prevention and/or treatment of caries or tooth erosion (e.g. WO 00/06108 A1 and WO 2010/042754 A2). Casein phosphopeptide amorphous calcium phosphate complexes (CPP-ACP) and CPP-stabilised amorphous calcium fluoride phosphate complexes have been described in combination with glass-ionomer cement as remineralising dental restorative materials, e.g., in WO 02/094204 A1.

Other calcium phosphate particles, e.g., comprising amorphous calcium phosphate, have also been used for remineralisation of demineralised enamel (e.g., Weir et al., 2012, J Dent Res. 91(10):979-984; Meyer et al., 2018. Open Dent J. 12:406-423).

Nanoparticles on starch basis have been described as useful for both caries diagnosis and treatment, e.g. incorporating calcium or phosphate ions for remineralisation of subsurface lesions (WO 2017070578 A1, WO 2019191456 A1, WO 2021062204 A1, Jones et al., 2024. Targeted enamel remineralization with mineral-loaded starch particles. JADA Foundational Science 3, 100041).

Amelogenin-based biomineralisation approaches for tooth repair have been described in the context of dentinal hypersensitivity, whitening or bleaching of teeth, and the treatment of caries in WO 2017/123986 A1, US 2014/0186273 A1 and US 2017/0007737 A1.

Remineralisation of demineralised enamel surfaces has also been attempted with nanoparticles of carboxymethyl chitosan/amorphous calcium phosphate particles (CMC/ACP) guided by chimeric peptides. The chimeric peptide required for this is rather long and comprises two functional domains, comprising an amelogenin-peptide that can transform ACP into hydroxyapatite, and one peptide that has been found to specifically bind to hydroxyapatite surfaces. An enamel like structure is formed, wherein CMC/ACP nanoparticles are degraded by NaClO and guided by the chimeric peptide into ordered and oriented arrays (Xiao et al., 2017. Dent. Mater. 33(11):1217-1228).

Enamel matrix derivatives or self-assembling peptides (SAP) have been shown to be effective in the remineralisation of subsurface carious lesions (Ruan et al., 2013. Acta Biomater.9(7):7289-97; Ruan et al., 2014. J Vis Exp. 10(89), doi 10.379151606; Schmidlin et al., 2016, J Appl Oral Sci. 24(1), 31-36; Alkilzy et al., 2018, Adv Dent Res. 29(1), 42-47; Brunton et al., 2013, Br Dent J, 215:E6; Kind et al., 2017, J Dent Res., 96(7), 790-797; Kirkham et al., 2007, J Dent Res., 86, 426-430). In WO 2014/027012 A1, a method for the targeted delivery of a SAP to a subsurface tooth lesion with the aim of remineralising carious lesions is described.

Self-assembling peptides such as those described in WO 2004/007532 A2 have been shown to be very successful in remineralisation of subsurface lesions (e.g., Kirkham et al., 2007), but remineralisation is not quick. It needs several weeks to months to become visible on dental radiographs or by change in appearance of the white spot lesion (Brunton 2013, Schlee 2017). For this reason, the self-assembling peptides alone are also not suitable for treatment of cavitated caries lesions, as, even in assembled form, i.e., as a hydrogel, they are likely to be removed from the cavity before the hydrogel becomes sufficiently remineralised and stable. WO 2021/005153 A1 describes SAP in the prevention and treatment of cavitated carious lesions, wherein the SAP are combined with a dental agent such as a glass-ionomer cement, wherein the incidence of secondary caries is reduced.

Self-assembling peptides have also been taught to be useful for regeneration of bone (e.g., WO 2004/007532 A2), with the same issue of slow mineralisation and the potential clinical drawback of lack of volume stability during said process.

WO 2022/084288 A1 teaches that remineralisation with SAP can be accelerated by combination with amorphous calcium phosphate and or solutions capable of immediately forming calcium phosphate precipitates.

In light of the state of the art, the inventors addressed the problem of overcoming at least some of these issues, advantageously, providing a composition and method for providing a targeted mineralisation of tooth lesions, in particular, subsurface caries lesions, and/or bone regeneration. This problem is solved by the present invention as disclosed herein, in particular, in the claims.

The present invention provides composition or kit comprising
a) a self-assembling peptide (SAP) comprising the amino acid sequence SEQ ID NO: 1,
b) calcium,
c) phosphate, and
d) a polymer in the form of particles having a diameter of 10-900.000 nm encapsulating a cargo selected from at least one of the self-assembling peptide (SAP), the calcium ions and the phosphate ions.

Encapsulation of at least one of the cargos, preferably, two or, optionally, all three of the cargos in polymer particles has advantages compared to a form in which the components are administered in a non-encapsulated form. Encapsulation provides a sustained release, in particular, of calcium and phosphate ions that can enable an improved and accelerated remineralisation process. This provision of ions occurs in a targeted manner, such that hydroxyapatite (HA) is quickly and efficiently formed in the subsurface caries lesions. Therefore, the compositions and kits of the present invention can be useful for remineralising tooth defects or bone defects. In particular, they are useful for treatment of caries, specifically, subsurface caries lesions.

In the context of the invention, phosphate can be comprised in the form of phosphate ions or in the form of covalently bound phosphate moieties that can release phosphate ions at any of pH 5 to 7, preferably, in the environment of a subsurface caries lesion. If this is not distinguished, i.e. if both are meant, the term "phosphate" is used herein.

The encapsulation of calcium (i.e., calcium ions) and/or phosphate, optionally, both in separate compartments, can also lead to their isolation from each other, and thus prevent premature precipitation into insoluble calcium phosphate.

This can advantageously be combined with SAP, which can be administered separately before administration of the particles, at the same time, or in the same composition or in the same particles.

In their early phase, active caries lesions, which progress towards cavitation and are demineralizing over time, typically have a slightly demineralized porous surface covering a still more demineralized subsurface lesion (ICDAS Code I-II). Subsurface demineralization may eventually cause collapse of the overlying tooth surface, which leads to cavitation. The white and rough surface of a subsurface caries lesion also leads to its designation as a "white spot". Beneath the surface, the lesions have an acidic pH, typically, about pH 5.5 or less. This allows for assembly of self-assembling peptides, as described herein, which have a high affinity to calcium and phosphate ions, and thus facilitate remineralisation. The constant supply of calcium and phosphate ions accelerates remineralisation in the subsurface caries lesions.

### Self-assembling peptides (SAP)

A composition or kit comprising a self-assembling peptide means that a single type of SAP or two or more, such as three, four or five etc., different types of SAP may be contained. For example, as described in detail below, the composition may contain two complementary peptides which self-assemble in combination.

SAP used in the present invention are peptides that are capable of forming three-dimensional scaffolds, thereby promoting tissue regeneration. They may assemble in one dimension to form beta-sheets, and higher order assemblies such as tape-like assemblies. Three-dimensional supramolecular structures of SAP can be formed, which have an affinity for/to calcium phosphate.

In the context of the present invention, SAP may be able to self-assemble by themselves, as is the case, e.g., for the peptides P11-4, P11-8, P11-2, P11-5 mentioned below, but they can alternatively be able to self-assemble in a combination of two SAP, as is the case, e.g., for the peptides P11-13/P11-14 and P11-28/P11-29, P11-30/ P11-31 mentioned below.

In the context of the present invention, SAP taught in WO 2004/007532 A1, US10/521,628, US12/729,046, US13/551,878, US 14/062,768, or WO2014/027012 A1, which are all fully incorporated herein by reference, are preferred. In particular, SAP having a net charge of +2 or -2 at pH 7.5 may be used in monomeric or assembled form.

SAP used in the present invention have the consensus sequence SEQ ID NO: 1, X1-X2-X1-X2-X1, wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine. Independently selected means that, e.g., X1 in positions 1, 3 or 5 of the sequence above can be different from each other. Of course, they can also be identical.

Preferably, SAP used in the invention comprise SEQ ID NO: 2, X1-X2-X1-X2-X1, wherein X1 is independently selected from the group consisting of glutamic acid and ornithine, and X2 is independently selected from the group consisting of tryptophan and phenylalanine.

In a further embodiment, SAP used in the invention may comprise SEQ ID NO: 3, X3-F-X1-W-X1-F-X1, wherein X1 is independently selected from the group consisting of glutamic acid and ornithine, and X3 is selected from the group consisting of arginine, glutamic acid and ornithine, wherein X3 preferably is arginine.

Furthermore, SAP used in the invention may comprise SEQ ID NO: 4 or, preferably, consist thereof: X4-X4-X3-F-X1-W-X1-F-X1-X4-X4, wherein X1 is independently selected from the group consisting of glutamic acid and ornithine, and wherein X3 is selected from the group consisting of arginine, glutamic acid and ornithine, and wherein X4 is independently selected from the group consisting of glutamine, glutamic acid, serine, threonine and ornithine. X3 preferably is arginine. Independently, X4 preferably is glutamine.

SAP of the invention may comprise SEQ ID NO: 5, or, preferably, consist thereof: Q-Q-R-F-X1-W-X1-F-X1-Q-Q, wherein X1 is independently selected from the group consisting of glutamic acid and ornithine.

Preferably, the SAP used in the present invention comprise or consist of a sequence selected from the consensus sequences listed in Table 1 below.

Most preferably, said peptides comprise the specific peptides listed in Table 2 or consist thereof. Of course, SAP assembling in combination with another SAP, e.g., as disclosed herein, may be formulated in one kit or in one composition.

Peptides of SEQ ID NO: 6, 9, 11, 12, 16 or 17 are particularly advantageous, e.g., as they can be used in relatively low concentrations, they are highly compatible with cells and have beneficial charge distribution. Advantageously, the SAP comprises SEQ ID NO: 6 or an amino acid sequence having at least 63%, at least 72% or at least 80% identity thereto. It can also have at least 90% sequence identity to SEQ ID NO: 6. Preferably, the SAP is P11-4 of SEQ ID NO: 6. A peptide consisting of the amino acid sequence of SEQ ID NO: 6 is preferred throughout the invention. P11-4 is monomeric at basic pH and starts to self-assemble at pH 7.5. It is thus assembled in the acidic pH of caries lesions.

SAP of the invention are, for example, 11 amino acids in length.

In another embodiment, the SAP comprises the sequence of SEQ ID NO: 9 or consists thereof (P11-8).

SAP may be modified peptides comprising an Ac-N-terminus and/or NH₂-C-Terminus, preferably, both. They can also be non-modified peptides. As non-blocked forms tend to start a deaminization reaction, the termini of all self-assembling peptides of SEQ ID NO: 1 are preferably blocked to increase stability. In particular, peptides of SEQ ID NO: 6, 9, 11, 12, 16 and 17 typically comprise an Ac-N-terminus and NH₂-C-Terminus.

**Table 1: Consensus sequences of preferred SAP**

| **SEQ ID NO** | **Peptide name** | **Sequence** | **Exemplary SAP** |
|---|---|---|---|
| SEQ ID NO: 1 | Consensus sequence 1 | X1-X2-X1-X2-X1, | P11-2, P11-4, P11-5, P11-8, P11-12, P11-13, P11-14, P11-17, P11-19, P11-20, P11-28, P11-29 |
| | | wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine | |
| SEQ ID NO: 2 | Consensus sequence 2 | X1-X2-X1-X2-X1, | P11-4, P11-8, P11-12, P11-13, P11-14, P11-17, P11-28, P11-29 |
| | | wherein X1 is independently selected from the group consisting of glutamic acid and ornithine, | |
| | | and X2 is independently selected from the group consisting of tryptophan and phenylalanine | |
| SEQ ID NO: 3 | Consensus sequence 3 | X3-F-X1-W-X1-F-X1, wherein X1 is independently selected from the group consisting of glutamic acid and ornithine, | P11-4, P11-8, P11-12, P11-13, P11-14, P11-17, P11-28, P11-29 |
| | | and X3 is selected from the group consisting of arginine, glutamic acid and ornithine, wherein X3 preferably is arginine | |
| SEQ ID NO: 4 | Consensus sequence 4 | X4-X4-X3-F-X1-W-X1-F-X1-X4-X4, | P11-4, P11-8, P11-12, P11-13, P11-14, P11-17, P11-28, P11-29 |
| | | wherein X1 is independently selected from the group consisting of glutamic acid and ornithine, | |
| | | and wherein X3 is selected from the group consisting of arginine, glutamic acid and ornithine, | |
| | | and wherein X4 is independently selected from the group consisting of glutamine, glutamic acid, serine, threonine and ornithine. X3 preferably is arginine. Independently, X4 preferably is glutamine. | |
| SEQ ID NO: 5 | Consensus sequence 5 | Q-Q-R-F-X1-W-X1-F-X1-Q-Q, wherein X1 is independently selected from the group consisting of glutamic acid and ornithine. | P11-4, P11-8 |

**Table 2: Preferred self-assembling peptides. Positions X1 are underlined**

| **SEQ ID NO** | **Peptide name** | **Sequence (One letter code)** | % **amino acid identity** to **P11-4** (ClustalW (2.1, standard parameters)) |
|---|---|---|---|
| SEQ ID NO: 6 | P11-4 | QQRFEWEFEQQ | 100 |
| SEQ ID NO: 7 | P11-2 | QQRFQWQFEQQ | 81.8 |
| SEQ ID NO: 8 | P11-5 | QQRFOWOFQQQ | 72.7 |
| SEQ ID NO: 9 | P11-8 | QQRFOWOFEQQ | 81.8 |
| SEQ ID NO: 10 | P11-12 | SSRFOWOFESS | 45.4 |
| SEQ ID NO: 11 | P11-13 | EQEFEWEFEQE | 72.7 |
| SEQ ID NO: 12 | P11-14 | QQOFOWOFOQQ | 63.6 |
| SEQ ID NO: 13 | P11-17 | TTRFEWEFETT | 63.6 |
| SEQ ID NO: 14 | P11-19 | QQRQOQOQEQQ | 54.5 |
| SEQ ID NO: 15 | P11-20 | QQRQEQEQEQQ | 72.7 |
| SEQ ID NO: 16 | P11-28 | OQOFOWOFOQO | 45.4 |
| SEQ ID NO: 17 | P11-29 | QQEFEWEFEQQ | 90.9 |
| SEQ ID NO: 30 | P11-16 | NNRFOWOFENN | 45.4 |
| SEQ ID NO: 31 | P11-18 | TTRFOWOFETT | 45.4 |
| SEQ ID NO: 32 | P11-26 | QQOQOQOQOQQ | 36.4 |
| SEQ ID NO: 33 | P11-31 | SSOFOWOFOSS | 27.3 |

The SAP preferably do not have restriction sites for the subject's endopeptidases. They also do not need to comprise a special recognition motif for cells.

Alternatively, or additionally to the pH, high ionic strength also leads to assembly of the SAP (Maude 2011. Soft matter 7: 8085-8098, Carrick 2008. Tetrahedron 63: 7457-7467).

The skilled person will know how to determine and measure the ionic strength of a solution. The ionic strength I is generally calculated according to the formula I = ½Σ zᵢ²bᵢ, wherein z is the valence factor and bᵢ is the molality [mol/kg{H₂O}] of the i^{th} ion concentration. The summation, Σ, is taken over all ions in a solution. For example, the ionic strength of a 150 mM NaCl solution is approximately 0.15 mol/L. This is also approximately the ionic strength of blood. The ionic strength of saliva present in the oral cavity is generally much lower, such as approximately 0.04 mol/L. In the context of the invention, ionic strength in the physiological range is considered to correspond to a ionic strength of 0.15 mol/L.

In one embodiment, in the kit or composition according to the present invention, the SAP is in predominantly monomeric form, e.g., at least 70%, at least 80%, at least 90% or essentially all of the SAP are present in a monomeric state. To this end, if the peptide assembles in a pH at or below 7.5, the pH of the composition may be above the pH wherein the peptide starts to undergo self-assembly (e.g., pH 7.5 for P11-4), preferably, 0.1 to 0.5 pH units above said pH, or more than 0.5 pH units above said pH. The pH may be buffered at that pH to avoid quick aggregation (e.g., with a non-ionic buffer such as Tris). It may be beneficial if aggregation, and formation of a hydrogel starts quickly after application to the caries cavity. Accordingly, the pH may be 0.1-1 pH units, preferably, 0.1 to 0.5 pH units above the pH at which the peptide starts to undergo self-assembly, without buffering. In one embodiment, the composition or kit may comprise dried (undissolved) peptide, e.g., obtainable according to WO 2014/027012 A1. Monomeric SAP is preferably used if a kit of the invention is supplied, comprising, in one compartment, monomeric SAP, preferably, in dry form, and in another compartment, the particles, which at least comprise calcium. The particles can be in solution, and optionally, the solution can be used before application to resuspend dry SAP and/or other components of the kit. The particles may also be in dry form. In another embodiment, monomeric SAP and particles comprising calcium in dry form may also be in the same compartment. In a further compartment, a solvent may be provided, which may e.g. be water (such as deionized or distilled water), or a solution comprising fluoride and/or phosphate ions. If the particles do not comprise phosphate, the solvent comprises at least phosphate ions, optionally, fluoride and phosphate ions.

In another embodiment, the kit or composition according to the invention comprises SAP in predominantly assembled form, e.g., at least 70%, at least 80%, at least 90% or essentially all SAP are in assembled form. The composition may also comprise a buffer at a pH stabilizing the assembled form. Assembled SAP typically forms a hydrogel.

In the context of the present invention, for treatment of subsurface caries lesions, assembled SAP can be used if it is contained in nanoparticles that are able to penetrate into subsurface caries lesions. The reason for using monomeric SAP for treatment of such lesions so far was that assembled SAP typically forms a constant hydrogel that would not be able to enter the sub-surface lesions, and would thus stay outside such lesions. However, according to the present invention, if the SAP does not form a constant hydrogel phase, but is part of nanoparticles that have a size small enough to enable transfer into subsurface lesions (i.e., less than about 1 µm, preferably, up to 500 nm, e.g., 10-500 nm, e.g., 50-500 nm, 100-500 nm, 200-400 nm or 250-300 nm), assembled SAP can advantageously also be used. This solves some problems associated with monomeric SAP, e.g., destabilization and aggregation during drying or storage, the requirement for the absence of water, low ionic strength etc.

Combinations of complementary SAP, e.g., of P11-4 and P11-8, provide a significantly faster assembly time resulting in a faster application and stability due to their attraction to each other.

In one advantageous embodiment, the SAP is encapsulated in the particles. Together with the particles, it is thus targeted to subsurface caries lesions and assembles in these. Advantageously, it is released from the particles due to their degradation and due to the SAP's affinity to the hydroxyapatite of the enamel. Said affinity can also contribute to targeting the particles to the subsurface lesions. If calcium and phosphate are also encapsulated in the particles, only one composition needs to be administered to the subject's teeth or oral cavity.

Alternatively, the SAP is not encapsulated in the particles, but in one composition with the particles. The SAP can for example form particles essentially by itself, in assembled form, preferably, nanoparticles small enough to enter subsurface caries lesions. It can also be monomeric SAP solution, obtainable from reconstitution (e.g., with water or an aqueous fluoride solution or a buffer), of dried monomeric SAP obtainable according to WO 2014/027012 A1. This can then be combined with nanoparticles comprising at least one of calcium ions and phosphate, optionally, both.

Optionally, the SAP can be part of a kit further comprising the particles, wherein the SAP can be, e.g., dried monomeric SAP obtainable according to WO 2014/027012 A1. For example, the SAP can be P11-4, such as Curodont^{®} Repair (vVardis, Switzerland). If the SAP is in dried form, it can be reconstituted (e.g., with water or an aqueous fluoride solution or a buffer), and applied to the surface of a tooth having a subsurface caries lesions, e.g., in a targeted manner and typically, after cleaning the tooth, wherein, optionally, cleaning includes removal of the pellicle. The SAP can also be administered to several, e.g., all teeth of a subject regardless of diagnosis of caries lesions, e.g., in the form of a mouthwash or toothpaste. Then, to accelerate and improve remineralisation of the lesion, a composition comprising particles encapsulating at least one of calcium ions and phosphate can be administered. This can also be administered directly to a subsurface caries lesion (i.e. to the enamel surface above a subsurface caries lesion) or to several, e.g., all teeth, e.g., in the form of a mouthwash, toothpaste or dental strip.

### Preferred characteristics of the particles

The particles of the invention formed by the encapsulation can be nanoparticles or microparticles.

Preferably, the particles are nanoparticles having a diameter of 10-999 nm, optionally, 10-700 nm or up to 500 nm. While the nanoparticles can be essentially round or round, they can also have other shapes. The diameter is preferably determined by optical microscopy, by SEM and/or as the Z-average size in dynamic light scattering (DLS). SEM is preferred. Alternatively, it can also be determined as the mean size in nanoparticle tracking analysis (NTA), DLS and NTA can be carried out as specified, e.g. in US 1,1666,515 B1.

Nanoparticles having a diameter of up to about 1 µm, preferably, up to about 500 nm can enter into subsurface caries lesions through nanopores. In a preferred embodiment, the nanoparticles thus have a diameter of 50-500 nm. Smaller nanoparticles may be able to enter still more easily. For example, nanoparticles can have a diameter of 50-350 nm, e.g., 100-200 nm. Larger particles can already partially degrade, e.g., under the influence of saliva, and may then be able to enter into subsurface lesions.

Alternatively, the particles are microparticles having a diameter of at least 1.000 nm, such as 1.000-900.000 nm. The diameter can be, e.g., 2.500-7.500 nm or 3.000-5.000 nm. Without intending to be bound by the hypothesis, while such particles may not be able to enter subsurface lesions directly, they may attach to plaque or to the surface of the enamel and provide their cargo to caries lesions by diffusion of the cargo through the pores. Particles of any size, regardless of their ability to enter subsurface lesions, may also be useful for remineralising cavitated caries lesions, e.g., in combination with glass-ionomer cement or composite fillings. Combinations with fluoride varnish may also be used both for subsurface and cavitated lesions.

The enamel of active carious lesions has a negative charge. The process of demineralization releases free ions, such as calcium ions and magnesium ions, many of which are cationic. Release of these ions appears to result in the remaining demineralized surface having a negative charge. Further, the pH inside subsurface lesions is acidic. If not defined otherwise, caries lesions herein are active lesions, as these are the lesions that require treatment.

Therefore, preferably, the particles bear at least one cationic (i.e., having a positively charged) surface region for associating the particles with a subsurface caries lesion in a tooth in an oral cavity of a subject. This allows selective association of the particles with active caries lesions. In particular, nanoparticles with a diameter that allows for diffusion into subsurface caries lesions may accumulate in such lesions and provide their cargo to the lesions. Optionally, the particles can have an overall net positive charge.

The electrokinetic potential of particles in colloidal dispersions is the zeta potential. It is caused by the net electrical charge contained within the region bounded by the slipping plane. The zeta potential is the potential difference between the dispersion medium and the stationary layer of fluid attached to the dispersed particle. A negative charge is generally associated with a positive zeta potential. It was found that particles having a positive zeta potential, e.g., having a positive zeta potential at pH 5.5 - pH 7.0 are particularly suitable for accumulating in subsurface caries lesions (WO2017070578A1, WO2019191456A1). For example, they can be administered as a diagnostic or therapeutic composition, e.g., in the form of a mouthwash or a rinse. The pH in the saliva of the mouth typically is about neutral, i.e., about pH 7. The particles may thus, in one embodiment, have a positive zeta potential at pH 7.0. The pH in the subsurface lesion is acidic, e.g., pH 5-5.5. Therefore, attraction of the particles to the subsurface lesions is improved if they have a positive zeta potential at the relevant acidic pH of pH 5.5 or less. The zeta potential may also be positive at both pH 7 and pH 5.5.

The positive zeta potential preferably is a zeta potential of +2 to +50 mV, e.g., a zeta potential of +2 to +30 mV, optionally, +2 to +20 mV. The positive zeta potential can also be a zeta potential of +2 to +5 mV. A compound or nanoparticle having cationic moieties or a net positive charge, for example of +2 mV or more, is attracted to the active lesion.

However, some studies with other positively-charged nanoparticles (for example gold nanoparticles and lipid nanoparticles) have found that particles with a higher positive charge, for example a zeta potential of +38 mV, had some toxic effects. While other studies have shown no toxic effect with such positively-charged nanoparticles, in certain aspects, nanoparticles used in accordance with certain aspects of the present disclosure have only a moderate positive charge, for example, less than or equal to about 30 mV or optionally less than or equal to about 20 mV.

The components or nanoparticles are optionally water-soluble or dispersible (WO2017070578A1, WO2019191456A1). A higher zeta potential, e.g., about 30 mV or more, can contribute to stability of a dispersion of particles.

Compositions or kits of the invention having a positive zeta potential can be administered in a targeted manner, e.g. to the enamel surface at a white spot, or in a non-targeted manner, e.g., as a mouth wash, a rinse, a toothpaste etc, regardless of the diagnosis of the presence of an active caries lesion. Because of the targeting characteristics of the particles, they will accumulate in active caries lesions, if present, and lead to their remineralization.

Particles having a different zeta potential, e.g., a neutral or negative zeta potential can also be used in the context of the invention. They can e.g., adhere to enamel, in particular to subsurface lesions, because of the SAP's affinity to hydroxyapatite of the enamel.

If the composition of the invention or the components of the kit of the invention are administered to a caries lesion, e.g., to a subsurface lesion, in a targeted manner, the surface charge and the zeta potential of the particles can also differ. For example, particles, e.g., nanoparticles, with a negative zeta potential at pH 5.5. to 7 may also be used.

Preferably, according to the invention, the particles are sensitive to a trigger leading to targeted release of their cargo. Targeted, in the context of targeted release, means that the release starts or takes place in the presence of a trigger. Said trigger is associated with administration to the oral environment, e.g., to saliva, and/or with localisation in a subsurface lesion, e.g., with acidic pH. For instance, the trigger ca be a pH of 5.5 or lower, i.e., the environment of a subsurface caries lesion. This has the advantage that the cargo is released in the lesion, and can thus facilitate remineralization in a targeted manner. The trigger can also be presence of saliva, e.g., an enzyme contained in saliva, such as presence of amylase. Release can also be multifactorial, e.g., due to the presence of water, e.g., hydrolysis, osmotic pressure, swelling or solubility of carrier material, optionally, in addition to other factors. In other embodiments, body temperature can lead to or contribute to release of the cargo.

Release of the cargo preferably does not mean that all cargo is released at the same time. Rather, a slow and sustained release of cargo is preferred, as it enables remineralization over a sustained period of time. Sustained release can mean, e.g., release over the course of several minutes to up to and/or even for longer than a month, e.g., at least 5 minute, at least 10 minutes, at least 20 minutes, at least 30 minutes, at least 1 hour, at least 2 hours, at least 4 hours, at least 6 hours, at least 12 hours, at least 24 hours, at least 2 days, at least 3 days, at least 4 days, at least 5 days, at least 6 days, at least 7 days, at least 10 days, or at least 14 days, or at least one month. Preferably, the cargo is released in less than a week, and remineralization of the subsurface lesion is complete in less than a week.

Further, not all of the types of cargo needs to be released at the same time. For example, either calcium ions or phosphate ions can be substantially released before the other, e.g., if the particles have a core and a shell with different components.

As explained above, if the particles are pH sensitive and release at least one of their cargo at a pH of 5.5 or lower, optionally, at least two of their cargos or all cargos, the cargo is provided at the place where it is needed, i.e., in subsurface lesions. If plaque is not removed before administration, the low pH there can also lead to release of cargo from particles accumulating in plaque.

This can be desired to reduce demineralization at plaque sites. It can also be prevented or reduced by removal of plaque before administration.

In another advantageous embodiment, the presence of amylase triggers release of at least one cargo, and the particles comprise a carbohydrate sensitive to amylase, optionally, starch. Amylase is present in saliva. WO2017070578A1, WO2019191456A1 describe excellent results with targeted release of cargo, such as calcium ions from starch particles in subsurface caries lesions.

### Polymers

The particles encapsulating the cargo are formed by at least one polymer. The polymer may belong to different classes of substances. Generally, all components of the composition are biocompatible, e.g. they are non-toxic. The polymer preferably also is biodegradable. The polymer may be selected from the group comprising a carbohydrate, a peptide, polyaspartic acid (PASP), poly(allylamine) hydrochloride (PAH), poly(N-isopropylacrylamide), poly(ethylene glycol)-block-poly(lactic acid), polyvinylalcohol (PVA), polyethyleneimine (PEI), poly(L-lysine) (PLL), poly(L-arginine), poly(amidoamine) (PAA), poly (amino-co-ester) (PAE), poly(2-N,N-dimethylaminoethylmethacrylate) (PDMAEMA), poly(4-vinylpyridine) (P4VP), polyesters, poly(acrylic acid), poly(methacrylic acid), a polyalkylene glycol, a methyl vinyl ether/maleic anhydride copolymer, gelatine, and combinations thereof. The particles can comprise only one polymer, or a combination of two, three, four or even more polymers. For example, it may comprise a combination of two carbohydrates, e.g., of alginate and chitosan.

It is noted that, while the self-assembling peptides, as peptides, are also polymers, they are not considered as the polymer in the form of particles having a diameter of 10-900.000 nm encapsulating a cargo in the context of the invention. Instead, in the context of the invention, the self-assembling peptides may be cargo of the particles, but they are in any case present in addition to the polymer.

The polymer may be a biopolymer, i.e., a polymer derivable from natural sources, optionally, in modified form. The biopolymer may be modified, e.g., to adapt characteristics such as solubility, chain length, charge (for example, it may be modified to comprise cationic moieties), pH, or to comprise pH modifying moieties and/or cargo.

Preferred polymers are carbohydrates. Carbohydrates, and particles formed by carbohydrates, can be easily modified to have the desired properties. The at least one carbohydrate may be, e.g., alginate, chitosan, starch, pectin, cellulose, carboxymethylcellulose, dextrin and/or dextran. The particles may comprise only one carbohydrate. Alternatively, two or more carbohydrates, e.g., two carbohydrates, can be combined, either homogenously, or in a core shell structure.

The carbohydrate can be alginate. Alginate is a naturally occurring polymer typically obtained from brown seaweed. It has been extensively investigated and used for many biomedical applications due to its biocompatibility, low toxicity, relatively low cost, and mild gelation by addition of divalent cations such as Ca²⁺. Alginate is an anionic (negatively charged) polymer due to its carboxyl groups, and the calcium ions are crosslinked within the alginate structure, forming a stable gel network. Thus, alginate is well suited to transport calcium as the cargo. If the carbohydrate is alginate, the particles thus comprise at least calcium as cargo, optionally, they further comprise SAP and/or phosphate, e.g., phosphate ions.

Alginate contains blocks of (1,4)-linked β-D-mannuronate (M) and α-L-guluronate (G) residues. Only the G-blocks of alginate are believed to participate in intermolecular cross-linking with divalent cations (e.g., Ca²⁺) to form hydrogels. The percentage of G (guluronic acid) content in alginate typically ranges from 30% to 70%, depending on the seaweed species from which the alginate is extracted. The composition (i.e., M/G ratio), sequence, G-block length, and molecular weight are thus important factors affecting the physical properties of alginate and its resultant hydrogels. Alginates are pH-sensitive, i.e., they dissociate at acid pH, e.g., at pH of 5.5 or less. Alginate particles can thus release their cargo in a targeted manner in the acidic environment of caries lesions, e.g., subsurface caries lesions.

The concentration of alginate in a solution prepared for generation of particles can e.g., be 0.01-3% w/w or 0.5-3% w/w, such as 1-2 % w/w. For nanoparticles, the concentration of alginate is typically lower than for microparticles. For preparation of alginates, these are dissolved in an aqueous solution, such as a buffer or water. The alginates can e.g. have a high G content, i.e., guluronic acid content (e.g., 60-70% G content), e.g., as derivable from species like *L. hyperborea.* It can have low to medium viscosity, comparable to KIMICA ALGIN IL-6G (viscosity at 20°C 30-60 mPas at 1%). Calcium concentration in solutions used for formation of particles can be, e.g., 0.01-0.5 M, such as 0.05-0.1 M.

Calcium alginate particles can be obtained, for example, by dripping the calcium solution into the alginate solution, or vice versa. The duration for which the particles formed remain in the solution bath may influence the formation of the cross-linking network and its strength, i.e., the cross-linking will be stronger if they remain in the solution longer. Accordingly, the time in which the particles release their cargo will also be longer. The size of the particles will also depend on the conditions of formation, primarily the concentration of alginate and calcium, as well as the dripping technique:
Macro Beads Formation occurs with high alginate concentration: When the alginate solution is relatively concentrated, dripping calcium solution typically forms macro beads. This is because higher concentrations lead to larger gelation zones and more extensive cross-linking, resulting in beads that are visible to the naked eye. The dripping technique also plays an important role: Using larger droplets (e.g., from a pipette or syringe without fine control) favors the formation of macro beads.

Nanoparticle Formation occurs with low alginate concentration: When the alginate solution is dilute and calcium is added in controlled conditions (e.g., through ultrafine droplets or slow addition under stirring), nanoparticles can form. The limited cross-linking and interaction at the molecular level favor smaller particle sizes. Rapid Mixing, e.g. high-speed stirring or ultrasonic methods can promote the formation of nanoparticles by dispersing the ions evenly before significant aggregation occurs.

The skilled person is aware of different options to prepare microparticles or nanoparticles.

For example, nanoparticles prepared according to any of the methods cited in Paques et al., 2014. Preparation methods of alginate nanoparticles. Advances in Colloid and Interface Science 209:163-171 can be used, Particles prepared according to Yu et al., 2008. Fabrication of nanospheres and versicles as drug carriers by self-assembly of alginate. J. Phys. Chem. C 112:16774-16778 can also be employed.

Vekatesan et al., 2017 (Preparations and Applications of Alginate Nanoparticles, Chapter 13, ISBN: 978-0-12-809816-5) also describe different preparation methods. Mukhopadhay et al., 2015 (pH-sensitive chitosan-alginate nanoparticles for efficient and safe oral insulin delivery. Int. J. Biol. Macromol. 72, 640-648), reported chitosan-alginate nanoparticles prepared by ionotropic gelation method with the average size of 100-200 nm. In another study, alginate-chitosan-tripolyphosphate nanoparticles were developed using ionic gelation method. The particles size (260-525 nm) was increased with increasing the molecular weight of alginate (4 to 74 kDa) (Goycoolea et al., 2009. Chitosan-alginate blended nanoparticles as carriers for the transmucosal delivery of macromolecules. Biomacromolecules 10(7), 1736-1743). Glycyrrhetinic acid-modified alginate nanoparticles release their cargo under acidic and neutral conditions (Guo et al., Functional alginate nanoparticles for efficient intracellular release of doxorubicin and hepatoma carcinoma cell targeting therapy. Int. J. Pharm. 451 (1). 1-11). Such nanoparticles can be modified not to comprise insulin, doxorubicin or other drug cargo. Instead, optionally, they may instead incorporate SAP, or they can be used in combination with SAP not comprised in the particles.

Phosphate, e.g., phosphate ions, can also be incorporated into alginate beads or alginate particles (used synonymously herein). However, as use of calcium is desired in the context of the invention, core-shell structures can be used to physically separate phosphate ions from direct interaction with calcium ions until release is desired. Such particles are for example disclosed in Singh et a., 2020. Environmental Geochemistry and Health 43: 2459-2482. In this approach, the alginate beads can, e.g., have an outer layer that is crosslinked with calcium ions to form a stable structure, while the inner core can contain phosphate, e.g., phosphate ions. This can help maintain stability by preventing direct contact between calcium and phosphate ions in the bead.

Matrix type beads or multicore beads (wherein several cores, i.e., at least 2, but optionally, 3, 4, 5, 6, 7, 8, 9, 10 or more cores are encapsulated in a matrix. Cores are typically identical, but can also be different, e.g. one comprising calcium and one comprising phosphate as cargo) or multishell beads (wherein at least two shells surround a core) or nanocapsules can also be used, wherein, preferably, calcium and phosphate are contained in different compartments of said beads (e.g., core and shell(s) are different compartments).

SAP, such as P11-4, can also be incorporated into the particles. Without intending to be bound by the hypothesis, the negatively charged groups within the SAP interact with calcium ions, which may promote the formation of hydrogel-like structures or nano-assemblies. In combination with alginate, which also forms cross-links with calcium ions, the interaction of calcium with P11-4 may enhance the stability of the network. This may enable a more controlled release of both calcium ions and P11-4 in the lesion environment, thus optimizing the remineralization process.

The particles can comprise alginate as the only carbohydrate, or they can comprise alginate in combination with a different carbohydrate, e.g., in combination with chitosan.

The at least one carbohydrate forming the particles can also be chitosan. Chitosan can optionally be the only carbohydrate in the particles. Chitosan is a linear polysaccharide composed of randomly distributed β-(1→4)-linked D-glucosamine (deacetylated unit) and *N*-acetyl-D-glucosamine (acetylated unit). It has antibacterial and antifungal properties. It is typically made by treating the chitin shells of shrimp and other crustaceans with an alkaline substance, such as sodium hydroxide. Commercially available chitosan has an average molecular weight ranging between 3800 and 20,000 Daltons and is 66% to 95% deacetylated. Chitosan is sensitive to slightly acidic pH, i.e., release of cargo can be triggered by the pH of subsurface caries lesions.

Chitosan is a polycationic biopolymer, i.e., it has positively charged amine groups along its chain. Further positive charges can be introduced by chitosan quaternization, which can be direct quaternization and indirect quaternization (Wikipedia on chitosan). Chitosan modified with quaternary ammonium groups is one of the most common cationic chitosan derivatives. Quaternized chitosan with a permanent positive charge has increased antimicrobial activity and solubility compared to normal chitosan.

Chitosan can also be phosphorylated to obtain phosphorylated chitosan, which is also biocompatible.

The positive charge of chitosan can accordingly be tailored to achieve the zeta potential charge of the particles, while, at the same time chitosan can serve as a carrier, e.g., for phosphates, e.g., phosphate ions.

Another factor plays a role in degradation of phosphate-crosslinked chitosan particles. At acidic pH (below pH 6.5), chitosan remains protonated, which makes the hydrogel formed, e.g. with phosphate, relatively stable. Thus, the hydrogel, and, accordingly, the particles, only degrade slowly, which leads to sustained release characteristics. Only below pH 4, there is a rapid dissolution. At neutral pH (6.5-7.5), chitosan starts losing its protonation, weakening the electrostatic interactions with phosphate ions. This can lead to gradual hydrogel degradation as the ionic cross-linking is disrupted. Degradation in this range is moderate and often used for sustained drug release in the art. At basic pH, above pH 7.5, chitosan is mostly deprotonated, which severely weakens interactions with phosphate ions. The hydrogel thus becomes unstable and degrades quickly. Phosphate cross-linked chitosan particles are thus well suited for sustained release. They are also advantageous in an embodiment of the invention in which release of the cargo affects the pH so that it becomes less acidic, as this then enhances degradation of the chitosan-based particles.

Chitosan nanoparticles can e.g., be prepared by a ionic gelation method, such as described by Sudheesh et al. 2013. Chitosan-based nanomaterials: A state-of-the-art review. International Journal of Biological Macromolecules 59, 46-58, e.g., according to Alonso et al., 1997, using sodium tripolyphosphate. The nanoparticles taught by Sawtarie, et al., 2017. Preparation of Chitosan/Tripolyphosphate Nanoparticles with Highly Tunable Size and Low Polydispersity, Colloids and Surfaces B: Biointerfaces, dx.doi.org/10.1016/j.colsurfb.2017.05.055 can also be used in the context of the present invention. Jiang et al., 2024 (Synthesis, characterization of chitosan/tripolyphosphate nanoparticles loaded..., Nature Scientific Reports 14:18754) disclose chitosan/tripolyphosphate nanoparticles that can be prepared without their herbicidal cargo, but already comprise phosphate as cargo, and can accordingly be used in the present invention.

Optionally, the particles comprise both chitosan and alginate. While homogenous mixtures of chitosan and alginate are possible, but non-homogenous particles can be advantageous, in particular, if the particles comprise both calcium ions and phosphate, e.g., phosphate ions. In that case, the calcium is typically used for cross-linking the alginate, and the phosphate, e.g., phosphate ions, is associated with the chitosan. The release of both ions in the presence of SAP associated with enamel hydroxyapatite leads to formation of new hydroxyapatite in an ordered manner that is accelerated compared to the absence of calcium and phosphate ions except from saliva.

The particles can have a core-shell structure. In one embodiment, the particle comprises a core comprising chitosan and a shell comprising alginate, e.g., a core comprising phosphate-cross-linked chitosan and a shell comprising calcium-alginate. Alternatively, the particle may comprise a core comprising alginate and a shell comprising chitosan e.g., a shell comprising phosphate-crosslinked chitosan and a care comprising calcium-alginate. The selection depends on the question if calcium (from alginate) or phosphate ions shall be released first. In deposition of natural hydroxyapatite, calcium is deposited first. SAP, e.g., P11-4 can be encapsulated, too. Preferably, it is at least comprised in the shell of the particles, as the SAP binds to the calcium phosphate of the enamel and then binds calcium and phosphate ions for remineralisation.

In one embodiment, the particles have a core-shell structure. The shell is pH-sensitive and comprises calcium and P11-4. In the slightly acidic environment, pH 5-6, i.e. in subsurface caries lesions, the outer shell of the nanoparticles will degrade and release the calcium ions and P11-4 peptide. The pH-sensitive shell could, e.g., be formed with (or from) chitosan and/or alginate, preferably, alginate. For instance, alginate is soluble at acidic pH in subsurface lesions, and thus said pH triggers the release of calcium and P11-4. This leads to enhanced remineralization and promotion of early events in enamel repair. The release of phosphate ions can be delayed such that it starts after calcium and P11-4 are released (e.g., until the majority of calcium and P11-4 are released, or until at least about 60%, at least about 70%, at least about 0% or at least about 90% of calcium and P11-4 are released. The core-shell particles can comprise the phosphate, e.g., phosphate ions in a more stable inner layer, for instance, an alginate core with a higher level of cross-linking than the shell. The core can also be a chitosan core crosslinked with phosphates. The core can alternatively comprise calcium phosphate, e.g., calcium phosphate nanoparticles or be a calcium phosphate core, e.g., an amorphous calcium phosphate core, or a phosphate-doped hydroxyapatite core.

The core of such a particle, e.g., a particle having an alginate shell that can release calcium and optionally, SAP such as P11-4, can also be a phosphate-modified starch core, as described below. The starch would only be exposed to the action of amylases after the core has been degraded. For example, the starch core could be phosphate cross-linked starch, e.g., Corn PO₄ PH "B" 9042, Agrana, Wien).

For nanoscale encapsulation, techniques such as emulsification or coacervation are often employed. Nanoscale encapsulation requires a very controlled process of particle formation. The size of the particles can be controlled by adjusting the concentration of the polymers, the cross-linking agent, in particular, calcium, and the conditions under which the particles are formed, such as pH, temperature, or ionic strength. Alginate and/or chitosan may be modified to allow for further cross-linking interactions.

Other techniques, such as nanoemulsification or solvent evaporation, may result in smaller particles of nanoscale to 1-100 nm. Encapsulated calcium and/or phosphate ions could be released gradually out of chitosan-alginate beads in response to pH and/or ionic strength. In bone tissue engineering, gradual mineral release may also take place.

In another embodiment, the carbohydrate is starch. Starch can be degraded in the presence of amylases, which are present in saliva. Thus, degradation is triggered after application and in the presence of enzymes from saliva. Starch can be the only polymer encapsulating the cargo. The starch can be amylopectin, and/or it can be amylose. It can be modified, e.g. such that the particles formed have the characteristics of the preferred particles described herein, e.g., the positive zeta-potential. Preferably, the particles are nanoparticles.

For example, the nanoparticles can comprise a plurality of molecules of starch polymer aggregated or crosslinked together to form the nanoparticles, the starch polymer bearing at least one cationic region for associating the nanoparticle with a subsurface portion of one or more carious lesions in a tooth in an oral cavity of the subject, wherein the at least one cationic region comprises a cationic moiety bonded with the starch polymer. The cationic moiety can comprise a tertiary or quaternary amine, such as a reaction product of glycidyl trimethyl ammonium chloride bonded to the starch polymer. The particle can be, e.g. a starch particle as described in WO 2017070578 A1. It preferably comprises a calcium and/or a phosphate-containing component, such as calcium glycerophosphate, dicalcium phosphate, tricalcium phosphate or calcium sodium phosphosilicate, casein phosphopeptide or phosphoprotein or calcium fluoride. For example, the starch particles can be phosphate cross-linked starch, e.g., Corn PO₄ PH "B" 9042, Agrana, Wien). It may further comprise a fluoride containing component. Optionally, the particle can also comprise a fluorescent moiety, which can facilitate control of treatment, but this is not required.

Starch particles having a size of 10-500 nm and a positive zeta potential at pH 7 and/or at pH 5.5 or less (preferably, both) are particularly preferred. Such nanoparticles that comprise phosphorous, as disclosed in WO 2019/191456 A1, can also be advantageously used. They may additionally comprise calcium and/or fluoride. The phosphate can be comprised in starch-phosphate compounds and/or dangling phosphate. It may e.g. be obtainable by crosslinking with a crosslinker comprising sodium trimetaphosphate. Preferably, the particles further comprise calcium.

In particular, the starch particles can be particles having a size of 150-300 nm prepared from cationic starch modified to incorporate phosphate and calcium, and optionally, fluoride, as used in Jones et al., 2024. Targeted enamel remineralization with mineral-loaded starch particles. JADA Foundational Science (2024) 3, 100041. Jones et al. showed *in vitro* proof of concept for sub-surface remineralisation with such particles. According to the present invention, this can be further improved and accelerated by the compositions and kits of the present invention.

Starch particles as described, or compositions comprising them can be obtained from GreenMark Biomedical Inc, East Lansing, MI. They have good targeting characteristics to subsurface caries lesions - in fact, when modified with a fluorescing agent, they can also be used for diagnosis of sub-surface caries lesions. In the subsurface lesions, due to the presence of amylase, the particles degrade over time and provide their cargo for remineralisation, which, in the context of the present invention, is mediated by SAP such as P11-4.

The starch particles, e.g., as disclosed in US 12,023,288 B2 or WO 2019/191456 A1 can be further modified to additionally comprise SAP, such as P11-4. Accordingly, the particles can comprise SAP, calcium and phosphate, e.g., phosphate ions. They can optionally also comprise fluoride ions. For example, in a preferred embodiment, SAP such as P11-4 can be mixed with the particles, which - without intending to be bound by the theory - can adhere to their surface because of the charge. They can also be incorporated during preparation of the particles.

However, these starch particles can also be combined with SAP that is not comprised in the particles, e.g., with SAP comprised in the composition, or with SAP in a separate composition (e.g., Curodont^{®} Repair or Curodont^{®} Repair Fluoride Plus, vVardis, Switzerland), optionally, wherein the SAP is in monomeric form. Indeed, separate sequential administration can be advantageous, as it may utilize existing products and technologies, and well-tested treatments can thus be administered to patients in an uncomplicated manner. For example, Curodont^{®} Repair Fluoride Plus can be combined with CrystLCare^{™} Biorestorative (GreenMark) either with fluoride or without. Curodont^{®} Repair can be combined with CrystLCare^{™} Biorestorative, Fluoride-free for a fluoride-free combination, if desired.

Pectin is another polymer that can be used in the context of the invention. Similar to alginate, pectin can be cross-linked by calcium salts, so calcium-pectin particles can be a good source of calcium. SAP can be physically entrapped in the particles. The gels formed are weaker than alginate gels. Thus, pectin can also be used as a shell surrounding a core of alginate or another stringer gel, both core and shell being cross-linked with calcium. The shell can comprise SAP.

Carrageenans are a family of natural linear sulfated polysaccharides that can also form a gel cross-linked with calcium, which is stronger than alginate gels. They can be extracted from red edible seaweed. Carrageenans contain 15-40% ester-sulfate content, which makes them anionic polysaccharides. They can be mainly categorized into three classes based on their sulfate content, wherein iota-carrageenan, which has two sulfate group per disaccharide and forms gels upon addition of calcium ions, is of particular interest in the context of the present invention.

Cellulose, e.g., derivatized forms thereof such as carboxymethylcellulose, is another polymer that can be used to form particles, and which can lead to targeted release in caries lesions, in particular, if the particles formed have the preferred size and zeta potential characteristics characterized herein which target them to subsurface caries lesions. Hydroxypropylcellulose or hydroxyethylcellulose can also be the polymer forming the particles of the invention. The cargo can be physically entrapped in the gel upon formation of gel particles. Alternatively, particles can be spray coated with the cargo, e.g., SAP.

Dextrin and/or dextran are alternative options for polymers.

Preparation methods for different polymer nanoparticles that can be used in the present invention are taught, e.g., in Zielinska et al., 2020. Polymeric nanoparticles: Production, Characterization, Toxicology and Ecotoxicology. Molecules 25;3731.

In one embodiment, the trigger for release of the cargo is pH, and the polymer is polymerized methacrylic acid. For example, particles formed with said polymer or coated with it would dissolve under slightly acidic conditions, such as in subsurface caries lesions. Once the outer layer has dissolved, calcium release starts, followed by phosphate ion release from the core. Alternatively, calcium and phosphate ions can be released at the same time. Phosphate ions can also be released first.

In another embodiment of the invention, the trigger for degradation of the particles and release of the cargo is body temperature. In this case, the polymers poly(N-isopropylacrylamide), poly(ethylene glycol)-block-poly(lactic acid), and/or gelatin are of particular interest., as they are capable of phase transition around body temperature, i.e., after the particles have transferred into subsurface caries lesions due to their advantageous targeting characteristics, namely, size and zeta potential that make them suitable for targeting subsurface caries lesions, as described herein. Such a carrier can complex, e.g., both calcium and phosphate, e.g., phosphate ions, preferably, in a core-shell structure. If desired, for sequential release is possible to design such particles to release calcium ions at lower temperatures, and then release phosphate ions at higher temperatures, due to variations of the polymer (e.g., polymer length). A core-shell structure may be easy to manufacture, and also allows for sequential release.

PVA, poly(vinyl alcohol), is an alternative polymer that can be used. PVA is a water-soluble material that has been widely used for immobilization of bioactive materials. PVA is innocuous for bioactive matter and possesses many attractive properties (i.e. hydrophilicity, reactivity, film formation, resistance to oxidation, and good mechanical properties). PVA is a hydrophilic polymer with abundant hydroxyl groups. PVA hydrogels can be prepared by physical crosslinking, chemical crosslinking, and/or radiation crosslinking. They have attracted considerable attention because of their low toxicity, high water absorption, good mechanical properties (i.e., high elastic modulus and mechanical strength), and good biocompatibility. In a study of enamel remineralization, PVA hydrogels were used as templates for synthesizing HAP crystals and to regulate the orientation and rate of crystal mineralization. In PVA hydrogels, the polymer chains of PVA are rich in hydroxyl groups, which show a partial negative charge to attract calcium. Therefore, PVA chains have multiple nucleation sites for HAP crystals and PVA hydrogels can be used as templates for HAP crystal mineralization (Liao et al., 2024, The application of hydrogels for enamel remineralization, Heliyon 10(13):e33574. In PVA hydrogels, the swelling decreased significantly with an increase in crosslinking density, thus improving the mechanical properties; however, the HAP crystal formation rate also decreased.

Calcium phosphate /PVA composite hydrogels and PVA calcium carbonate nanoparticles are known in the art (e.g., Timofejava et al.,2017. European Polymer Journal 95:547-565; Jahani et al., 2020. Polymers 12(10):2179) and such nanoparticles can be used in combination with SAP, e.g., as a kit to improve remineralization.

PVP, polyvinylpyrrolidone, can also be used to form particles encapsulating at least one of the cargos of the invention.

Throughout the invention, the particles can comprise one polymer only. In this context, the SAP is not considered a polymer. Alternatively, the particles can comprise at least two polymers, preferably two polymers, wherein SAP is not considered (or counted as) a polymer. If the particles comprise two polymers, a homogenous mixture is possible. However, a structure with different compartments, e.g., a core-shell structure (or alternatively, a matrix structure or a multicore structure, is preferred in this context, as it can keep calcium and phosphate separate and prevent premature formation of calcium phosphates, which are typically insoluble. If there are two polymers, for example, two carbohydrates can be combined, such as alginate and chitosan. Alginate, which is particularly well suited to be a source of calcium ions in the form of calcium-crosslinked alginate, can for example be used in the shell, which can optionally further comprise SAP, e.g., P11-4. The core may comprise chitosan and phosphate, and, optionally, also comprise calcium. Alternatively, the core can comprise starch, e.g., phosphate modified starch, and the shell may comprise another carbohydrate polymer, e.g., alginate crosslinked with calcium.

The particles may also comprise two shells, e.g., the outer shell comprising SAP. However, it is easier to prepare nanoparticles having less than two shells.

Encapsulation in the context of the invention does not require that the polymers completely encase the cargo molecules and/or shield them from the outside, e.g., from saliva or enamel surface. Cargo can also be present, at least partly, on the surface of the particles. Cargo molecules are often at least noncovalently associated with polymer molecules, and they can also be covalently associated.

In the form of particles having a diameter of 10-900.000 nm, the polymer encapsulates a cargo selected from at least one of the self-assembling peptide (SAP), e.g., P11-4, the calcium ions and the phosphate, e.g., phosphate ions or covalently bound phosphates.

### Stabilisation of nanoparticles

Aggregation of nanoparticles, in particular, of nanoparticles up to 500 nm, can reduce their effectiveness. Stabilisation measures can therefore increase the stability of nanoparticle size dispersion and function during storage and use.

Physical, chemical, and surface modification techniques may be used to stabilize nanoparticles. These most essential processes involve steric stabilization (e.g., with polymer layers like PEG or PVA) or electrostatic stabilization (e.g., polyelectrolytes or pH adjustment).

Particles with zeta potentials of +30 mV or more or of -30 mV or less are normally considered stable. Adjusting pH to maintain surface charge (e.g., at +30mV or more zeta potential) may prevent nanoparticle aggregation. As already explained, a positive zeta potential is preferred in the context of the invention.

It is also possible to add salts at appropriate concentrations to screen excessive repulsion without causing instability. Of course, if SAP in monomeric form are in contact with said salts, e.g., in the shell of a core-shell nanoparticle, the ionic strength should be carefully chosen to allow the SAP to maintain their monomeric form.

If the particles have a density greater than the dispersant, even though they are dispersed, they may still eventually sediment. Dispersal, e.g., by shaking, pipetting, mixing and/or sonication before application can help to form a dispersion again. The medium for dispersion can also be chosen to delay or reduce or prevent sedimentation, e.g., the dispersal medium can be in gel form, such as a Carbopol or hydroxypropyl cellulose. Dispersion in oil and/or wax (e.g, medium chain triglycerides (MCTs) or sesame oil may alternatively reduce Van der Waals forces, minimizing aggregation.

Surface modification is a very effective method of stabilizing polymeric nanoparticles, because it improves colloidal stability, biocompatibility, and functionalization capacity. The following are the main strategies, divided based on their mechanism of stabilization:
- Addition of surfactants and/or emulsifiers: surfactants (e.g., Pluronic, Tween 80, SDS) may be added to the composition comprising the nanoparticles to prevent aggregation through steric or electrostatic repulsion.
- Polyelectrolytes: coating of particles with charged polymers (for example, PVA, PEG, or chitosan) stabilizes nanoparticle dispersion using electrostatic or steric stabilization. Chitosan for example stabilizes using hydrogen bond and ionic interaction.
- PEGylation: Polyethylene glycol (PEG) may enhance colloidal stability by preventing protein adsorption and aggregation. Optionally, polymers used in the surface, e.g., the coating, of the nanoparticles, can be PEGylated. Alternatively, PEG can form a coating.

Accordingly, in one embodiment, the particles are nanoparticles and comprisean additional hydrophilic, e.g., charged, polymer (e.g., PEG), a surfactant or polyelectrolyte as particle coating, for enhancement of the colloidal stability of nanoparticles (< 500 nm). Steric hindrance can then reduce or prevent aggregation.

Polyelectrolytes are polymers composed of macromolecules in which a substantial portion of the constitutional units contains ionic or ionizable groups, or both. In water, the polyelectrolytes are charged. Polyelectrolytes that can be used are, e.g. pectin, carrageenan, alginates, PAA (polyacrylic acid), or carboxymethyl cellulose.

### Inducing a pH change

In one embodiment of the invention, release of cargo from the particles induces a pH change in a subsurface lesion such that the pH becomes less acidic, preferably, such that the pH in the subsurface lesion changes to pH 6-10, optionally, pH 6-8. An increased pH - compared to the normal pH in subsurface lesions, helps to shift the balance towards hydroxyapatite formation.

To allow for enhanced remineralisation, the SAP should stay assembled. It was known in the art that, if the SAP is P11-4, the SAP stays assembled below a pH of 7.5. Thus, there is no risk that the SAP disassembles prematurely if the pH is increased up to pH 6-7.5, e.g., pH 6-7.4. However, the inventors found that P11-4 also stays assembled, which mediates remineralisation under higher pH conditions, if the supply of ions is sufficiently high, e.g., under conditions providing a high ionic strength, e.g., upon administration with the particles of the invention For example, at a ionic strength corresponding to 130 mM NaCl, P11-4 stays assembled up to pH 10 or more (Carrick et al, 2007.Tetrahedron 63: 7457-7467). Of note, it is not required for this that the ionic strength is changed in the total oral cavity, but only in the microenvironment of the subsurface caries lesion where the cargo affects both ionic strength, by release of calcium and phosphate ions, and, in this embodiment, pH. Further, the binding of P11-4 to the calcium and phosphate also shifts the balance towards the assembled state, i.e., if assembly and remineralisation start at a lower pH, they can also continue at a pH higher than the pH at which the SAP normally disassembles.

Thus, in this embodiment, at least one cargo, in particular, at least one of calcium and phosphate source, are pH active agents, e.g., they can be buffers. Optionally, both calcium and phosphate source can be pH active agents, e.g., buffers. As described below, different calcium and/or different phosphate sources can be combined to achieve the desired pH and the desired release characteristics.

In a core-shell particle, for example, the inner layer might be designed to release phosphate ions only at higher pH conditions, for instance, about pH 7. To this end, a polymer that degrades better at a non-acidic pH may be used, e.g., cellulose, poly(β-amino esters), or PEG.

In other embodiments, the release of cargo and the degradation of the polymer particles do not significantly affect the pH of the subsurface lesion.

### Calcium and phosphate

The kit or composition of the invention comprises calcium, i.e., Ca²⁺ ions. Various calcium sources can be used, which provide calcium ions at a pH of about 5.5 or less or up to pH 7.5. Calcium can be derived, e.g., from calcium chloride, calcium hydroxide, calcium carbonate, calcium sulfate, calcium nitrate, calcium acetate, calcium oxide or calcium phosphate (e.g., ACP). Preferably, the calcium salt from which the calcium ions derive is Ca(C₂H₃O₂)₂, CaCl₂, Ca(OH)₂, CaCO₃, and/or calcium lactate.

Preferably, calcium salt that is well soluble in water is used, e.g., a calcium salt having a solubility in water (g/100 mM at 25°C of at least 7 g/mL, e.g., at least 30 g/mL, e.g., calcium chloride or calcium acetate. Such soluble calcium salts can be combined with less soluble calcium salts if a more sustained delayed release is desired.

If sustained release, e.g., over the time of 30 min or more, 1 h or more, 2 h or more, 4 h or more, 6 h or more, 12 h or more, 24 h or more, 2 days or more, 3 days or more, 4 days or more or a week or more is desired, it is preferred to use, instead of or, preferably, in addition to a calcium salt with good solubility such as calcium chloride, one or more calcium salts that do not have a good solubility, and are thus less likely to immediately react with phosphate ions and can thus prevent unwanted precipitation of calcium phosphate, e.g., outside the subsurface lesions. For example, calcium carbonate or calcium lactate or calcium phosphate can be used in this context.

Of course, sustained release can also be mediated by the characteristics of the polymer. Slow degradation of the polymer also leads to a sustained provision of the cargo to sustain remineralisation over an extended period of time, preferably, over the time of 30 min or more, 1 h or more, 2 h or more, 4 h or more, 6 h or more, 12 h or more, 24 h or more, 2 days or more, 3 days or more, 4 days or more or a week or more.

CaCl₂ is a calcium salt that can easily be used in the context of the invention, as it does not form precipitates and is well soluble in water, so it releases calcium ions quickly. Due to its advantageous solubility characteristics and lack of toxicity, calcium chloride is well characterized for cross-linking of carbohydrate polymers such as alginate, chitosan, carragenaan or pectin. Calcium chloride can also be used for encapsulating calcium in starch particles. Calcium chloride does not affect the pH. It can be used as the only calcium source. In this case, a delayed release can be achieved because of the slow degradation of the particles, which thus slowly release calcium. Optionally, delayed release can be achieved by addition of a less well soluble calcium salt.

Ca(C₂H₃O₂)₂, calcium acetate, has a moderate water solubility (about 37.4 g/100 mL water at pH 7). It thus still effectively releases calcium. Solubility is enhanced in mild acidity. Calcium acetate has a slightly basic pH, and dissolving calcium acetate increases the pH compared to the pH of a typical subsurface lesion of about pH 5.5. The pH can be increased with calcium acetate up to pH 8. Calcium acetate can thus be used if a modification of the pH through administration of the particles is desired to further improve remineralization.

Ca(OH)₂, calcium hydroxide, is a calcium salt that is strongly alkaline. pH can be increased up to 12.4. Calcium hydroxide however only has limited solubility in water even in acidic conditions, and thus the practical increase in pH will be more limited. Calcium hydroxide is preferably used in combination with other calcium compounds, most preferably also in combination with other pH-active agents.

Another interesting calcium salt that can be used in the context of the invention is CaCO₃. Calcium carbonate has a low solubility in water at pH 7, but solubility is better under acidic conditions. It can, when dissolving, release calcium and CO₂. CO₂ is soluble in water, and can form carbonic acid, but the majority of CO₂ remains in gaseous form and thus does not affect the pH. CO₂ can diffuse out of subsurface caries lesions through the pores. In one embodiment, CaCO₃ is not used to avoid gas formation.

Calcium lactate is another calcium source with low-moderate solubility in water, which is preferably used in combination with a better soluble calcium salt. The solubility of calcium L-lactate in water increases significantly in presence of d-gluconate ions, from 6.7 g/dl at 25°C to 9.74 g/dl or more. It can thus be used in the form of calcium lactate gluconate. If good solubility is desired, pure isomers are used, as the racemic form is less soluble. Hydrated forms have still better solubility, e.g., calcium lactate pentahydrate has solubility in water of or 79 g/L at 25°C. In aqueous solution, calcium lactate partially dissociates into calcium ion, which can contribute to remineralisation, and lactate ions, which make the solution mildly alkaline. Calcium lactate can thus be used if it is desired that the pH is rendered less acidic by release of the cargo. For example, a 0.01 mol/L calcium lactate solution has a pH of 7.4 to 7.6. Calcium lactate can crosslink sodium alginate to form the particles used in the invention.

One of the components of the kit or composition of the invention is phosphate. The phosphate can e.g., be in the form of sodium tripolyphosphate (TPP), sodium hexametaphosphate (SHMP), sodium phosphate (Na₃PO₄), potassium phosphate (K₃PO₄), disodium phosphate (Na₂HPO₄), monosodium phosphate (NaH₂PO₄), sodium pyrophosphate (Na₄P₂O₇), amorphous calcium phosphate (ACP), glycerol phosphate and/or covalently linked phosphate groups. The phosphate should be phosphate ions (preferably, PO₄³⁻, e.g., HPO₄²⁻, H₂PO₄⁻) or be able to release phosphate ions in the environment of a subsurface lesion.

Sodium tripolyphosphate (TPP) has a solubility (in water at 25°C of 15-20 g/100 mL. The pH of a saturated solution is 9-10, and phosphate ions are best released at neutral to basic pH. Thus, TPP is particularly suited as a phosphate source in the embodiment in which release of the cargo changes the pH to be less acidic.

Sodium hexametaphosphate (SHMP) typically is a mixture of metaphosphates comprising hexametaphosphate, which can also be referred to as sodium polymetaphosphate. It can also be pure sodium hexametaphosphate. It is highly soluble at about 60 g/100 mL (in water at 25°C). The pH of a saturated solution is 6.5-7.5, and it releases phosphate ions at acidic to neutral pH. It is thus well suited for application in the present invention. In particular, it can be used in the embodiment in which release of the cargo changes the pH to be less acidic. It is preferably combined with another salt rendering the pH more alkaline if a higher pH shift is desired.

Sodium phosphate (Na₃PO₄) also has a good water solubility at 87.8 g/mL (25°C). A saturated solution is alkaline at 11.5, and phosphate ions are mainly released at basic pH. It may be used in the context of the present invention in combination with other phosphate compounds.

Disodium phosphate (Na₂HPO₄) is soluble in water up to 7.7 g/100 mL at 25°C). The pH of a saturated solution is 8.5. Phosphate ions are best released at neutral to basic pH.

Monosodium phosphate (NaH₂PO₄) is well soluble in water at 25°C at 59.9 g/mL. The pH of a saturated solution is 4.5. Phosphate ions are best released at acidic to neutral pH. A combination of disodium phosphate and monosodium concentrate can be easily used to obtain a desired pH.

Amorphous calcium phosphate can be converted into hydroxyapatite, in particular, at neutral pH. In one embodiment, the particles of the invention do not comprise ACP and only comprise calcium and/or phosphate salts having a solubility better than ACP (at 25°C and neutral pH).

Glycerol phosphate is highly water soluble at 70-100 g/mL (25°C). It can release phosphate ions at pH 6-8. It is thus also particularly suited for the embodiment in which release of the cargo changes the pH to be less acidic.

TPP, SHMP, ACP and glycerol polyphosphate easily form chitosan crosslinks. Moderate cross-linking also occurs with sodium phosphate and monosodium phosphate as well as sodium pyrophosphate. Limited crosslinking is possible e.g., with sodium phosphate, which is therefore preferably used in combination with other agents that can crosslink chitosan, if chitosan is used for encapsulating the phosphate.

Instead of or in addition to using well-soluble sources of phosphate ions, phosphates not having a good water solubility can be employed, e.g., calcium phosphate nanoparticles (e.g. having a size of 1-50 nm, such as 10-20 nm) can be encapsulated or used as part of the particle matrix. These nanoparticles are less reactive than particles with free phosphate ions. They can be embedded in alginate particles, e.g. nanoparticles, offering a stable structure that still allows for controlled release of phosphate and calcium over time.

In one embodiment, the composition of the invention comprises both calcium and phosphate ions. Preferably, calcium and phosphate ions are released in an atomic Ca/P ratio of 1.5 to 3 e.g. 1.7 to 2.5 over the time the particles of the invention degrade. The rate of supply can be different. The concentration of each ion e.g. Ca and Phosphate generated by degradation of the particles of the invention in the subsurface lesions can be, e.g., 1-300 mM, 50-150 mM to 100-130 mM.

In the composition of kit of the invention, e.g., in the particles, calcium ions and phosphate ions can optionally be comprised each at least in the form of a salt having a solubility in water at 25°C and pH 7 of at least 7 g/100 mL. As discussed above, this can optionally be combined with less soluble salts such that release is maintained over a longer time.

In another embodiment, the phosphates are covalently bound to the polymer. Phosphate ions can be released e.g. at pH 7.5 pr less, e.g., pH 5.5 or less. The phosphate can be comprised in polymer-phosphate compounds, phosphoproteins and/or dangling phosphate. Such compounds are e.g. disclosed in WO 2019/191456 A1 for starch as a polymer. The phosphate can be an ester of a phosphoric acid with the polymer, e.g., an ester with orthophosphoric acid.

In one embodiment, both calcium and phosphate are encapsulated in the particles. They can be substantially homogenously mixed in the particles. Alternatively, the particles can be structured to release the majority of the calcium ions before the majority of phosphate ions. For example, the particles can comprise a shell comprising calcium ions and a core comprising phosphate (and optionally, calcium ions). In another embodiment, both calcium ions and phosphate are encapsulated in the particles and the particles are structured to release the majority of the phosphate before the majority of calcium ions. For example, the particles can comprise a shell comprising phosphate and a core comprising calcium (and, optionally, phosphate).

### Fluoride and other agents

The composition or kit of the invention may further comprise fluoride ions, wherein the fluoride ions are in solution or in the form of a soluble fluoride salt. Advantageously, fluoride ions were shown to lead to direct formation of HA crystals under conditions that in the absence of fluoride would lead to formation of calcium phosphate having a lower Ca/P ratio. Fluoride ions e.g., provided in the form of NaF can be incorporated in the particles, or in a solution comprising the particles, or in an aqueous solution for dissolving SAP and/or the particles in case these, or one of these is/are in dry form. Fluoride shifts the balance of the reaction towards direct formation of calcium phosphate having a high Ca/P ratio or more than 2, i.e., HA.

A soluble fluoride salt may be, e.g., NaF, NH₃F, MgF₂, SrF₂, Na₂PFO₃, SnF or a mixture thereof, optionally, NaF. NaF or NH₃F are routinely used in dental care products such as toothpastes.

They may be comprised in the composition comprising the particles, wherein said composition optionally further comprises the self-assembling peptide, calcium and phosphate. The fluoride ions can optionally be encapsulated in the particles.

The fluoride ions alternatively may be in a separate composition from the particles. For example, the fluoride ions can be part of an aqueous solution comprised in a compartment of the kit separate from the particles. This solution can be mixed with the composition comprising the particles before administration, e.g. a dry composition comprising the particles can be taken up in such a solution.

In one embodiment, the fluoride is administered sequentially after the composition comprising the particles, e.g., in the form of a fluoride varnish.

In one embodiment of the invention, the composition and the kit do not comprising fluoride ions. Some subjects have doubts about health hazards of fluoride. For such subjects, fluoride-free compositions and kits can be used without giving rise to doubts, and they can still effectively induce remineralisation.

In one embodiment, the particles further contain a fluorescent agent. Accordingly, they cannot only be used for treatment of subsurface caries lesions, but simultaneously, for diagnosis thereof. The fluorescent agent can e.g., be fluorescein or a derivate thereof such as fluorescein isocyanate or a reaction product with the polymer. Particles comprising fluorescein, are e.g. known from WO2017070578A1. The fluorescent agent can also be, e.g., riboflavin, which, as a vitamin, is advantageous from a toxicity standpoint.

Certain surfactants or stabilizers can be incorporated into the formulation to help maintain the integrity of the particles and prevent premature precipitation of calcium phosphate. For example, surfactants like polysorbates or Tween can help stabilize dispersion of ions within the beads and prevent them from reacting too early. Additionally, anti-crystallization agents or stabilizing proteins like albumin could be used to prevent calcium phosphate crystals from forming prematurely within the bead structure.

### Compositions and kits

In one embodiment of the invention, a composition comprising SAP, phosphate and calcium and particles encapsulating at least one cargo is provided. Preferably, all three cargos, and optionally, fluoride, are encapsulated. As explained herein, calcium and phosphate can optionally be contained in shell or core of structured particles, respectively. SAP are at least contained in the shell of core-shell particles, preferably also in the core.

The SAP can be monomeric, in particular, if the composition does not comprise water, or if the SAP are encapsulated in the particles in a way that prevents their assembly, e.g., in a compartment with low ionic strength (e.g. below 0.15 mol/L) and pH above their assembly point, e.g., above pH7.5 for P11.4.

However, as explained herein, assembled SAP can also be used as a part of nanoparticles which are themselves small enough to penetrate into the pores of subsurface lesions.

Alternatively, calcium and phosphate may be encapsulated, and SAP may be provided in non-encapsulated form, e.g., mixed as a dry powder with dry particles. In that case, a solution for dissolution or dispersion of the dried product can optionally also be provided in the form of a kit (e.g., water, such as deionized water or distilled water). After dissolution or dispersion, the resulting composition can be administered.

The invention thus provides a kit comprising a dry composition and a liquid composition, preferably, water or an aqueous composition. The dry composition can comprise both SAP (not encapsulated) and particles. It can also comprise particles that encapsulate SAP and calcium and phosphate. In one embodiment the particles and/or the SAP (preferably both) are associated with a solid support selected from the group comprising a sponge or pad or a film. The solid support may be from any suitable material, e.g., from cotton or a polymer, e.g., as described above. It may also be from a synthetic material such as PVA. In this embodiment, the solid support preferably is not suitable for being dissolved in the solution for dissolution or dispersion of the dried product.

The solid support can preferably be used for transferring the solution or dispersion or the product to the surface of at least one tooth, e.g., having a subsurface caries lesion. The solid support can be porous. It can be, e.g., a sponge. It can also be a pad or film. The solid support can e.g., be prepared by dip coating or spray drying, optionally, liquid bed spray drying, with particles and/or SAP. If the solid support comprises both particles and SAP, they can be applied sequentially or at the same time. They can e.g., be administered each to a different side of the support. They can also be administered to the same side sequentially.

In one embodiment, the particles comprise calcium (e.g., in the form of alginate particles) and do not comprise phosphate. They may optionally also comprise SAP such as P11-4. SAP can also be provided separately. Phosphate can be provided, e.g., as a solution or one or more phosphate salts. It can also be provided in encapsulated form e.g., in liposomes. These liposomes advantageously have a size that also allows them to diffuse into subsurface caries lesions. After administration to a subsurface caries lesion, the liposomes can release their cargo in the lesion, e.g., because of the action of a lipase comprised in saliva.

The composition preferably is a dental care product such as a mouth wash, a toothpaste, a tooth mousse, a solution for targeted application to a subsurface caries lesion, a chewy product or a film.

For example, the composition can be a mouth wash. As described above, it has been shown that particles from a mouthwash having the characteristics described above, in particular, the preferred size and positive zeta potential assemble in subsurface caries lesions and can even, if additionally loaded with a fluorescent component, be used for diagnosis. In the present invention, this is employed for provision of calcium and phosphate encapsulated in the particles which synergistically interact with SAP to enhance and accelerate remineralization of subsurface lesions. Preferably, the mouthwash is applied after brushing the teeth to reduce plaque and to enable access to the enamel.

A paste or gel, optionally, a toothpaste can be more concentrated than a mouthwash, brings the components of the composition into close contact with the tooth surfaces, and can be regularly used by a subject as part of the daily dental cleaning routine. A toothpaste may also comprise two compositions that are not homogenous, e.g., in the form of striped toothpaste, e.g., one composition comprising calcium and the other phosphate. Alternatively, a tooth mousse or tooth powder can be used.

The composition can also be a chewy product selected from the group comprising a chewing gum, a toffee, a chew toy, a lozenge, a tablet, a powder, a putty, granules or a candy. Chewy compositions typically comprise a base, e.g., a chewing gum base in addition to the particles. The base can comprise particles encapsulating calcium and phosphate and, separately, dry monomeric SAP. The SAP can also be co-encapsulated in the particles. The chewy composition preferably is substantially water-free, i.e., it does not comprise sufficient water to allow for aggregation of SAP, as disclosed e.g., in WO 2017/202940 A1.

The composition of the invention can also be a film for oral application, e.g., a film that dissolves over time after application to a tooth surface, wherein the film comprises the particles. These particles may comprise, e.g., calcium, phosphate and SAP. Alternatively, the SAP, e.g., in monomeric form, may be dispersed in the film base in which, further, particles comprising calcium and phosphate are dispersed.

The film (which can also be designated a strip or a biomembrane) may e.g., comprise a polysaccharide (e.g., as described above, collagen or gelatine). The film may alternatively or additionally comprise a synthetic material selected from the group comprising Teflon, or a synthetic polymer as described above, e.g., PVA and PLGA, and a combination thereof. The structure of the film is typically looser than the structure of the particles, i.e., the film dissolved on the tooth surface under conditions at which the particles do not yet degrade. Preferably, the particles substantially start degrading after entering into the subsurface caries lesion.

The film comprising the particles and/or SAP (preferably, both) can, e.g., be prepared by dip coating or spray drying the film, optionally, liquid bed spray drying. If the film comprises both particles and SAP, they can be applied sequentially or at the same time. They can e.g., be administered each to a different side of the film. They can also be administered to the same side sequentially.

The composition of the present invention can also be a wax stick. The wax can be natural, e.g., beeswax or synthetic, e.g., stearin. The wax can also comprise an oil to reduce viscosity, e.g., almond oil, olive oil or sesame oil.

Such compositions, like a mouthwash, can be used to treat and prevent (i.e., reduce incidence of) subsurface caries lesion without requiring their prior diagnosis.

In contrast, the composition of the invention can also be a solution for targeted application to a subsurface caries lesion. It is advantageously applied after cleaning of the tooth surface, preferably, after removal of plaque. Pellicle can also be removed, e.g., using pumice, prophy paste, air polishing or sodium hypochlorite. The tooth surface can be etched, using e.g., 35% phosphoric acid for 20 seconds. After rinsing and drying, the composition of the invention can be applied. The protocol can correspond to application of Curodont^{®} Repair Fluoride Plus.

Alternatively, the invention provides a kit having separate components. The kit comprises at least two optionally, three components. Some options are specified in the table below:

| SAP | calcium | phosphate | Additional component | example |
|---|---|---|---|---|
| Separate, optionally, in monomeric and dry form | Encapsulated in particles | Encapsulated in particles | Optionally, water or fluoride solution | e.g., combination of Curodont^{®} Repair (with or without fluoride) and starch-based particles, e.g., CrystL-Care^{™} Biorestorative |
| Separate, optionally, in monomeric and dry form | Encapsulated in particles, e.g., calcium alginate particles | Phosphate solution, optionally, with fluoride for dissolving SAP and, optionally, particles | - | e.g., combination of Curodont^{®} Repair (with or without fluoride) and calcium-alginate-based nanoparticles |
| Encapsulated in particles | Encapsulated in particles, e.g., calcium alginate particles | Separate phosphate solution optionally, with fluoride, for dissolving particles | - | |
| Encapsulated in particles | Calcium in separate solution | Encapsulate in particles | - | e.g., phosphate-cross-linked chitosan particles comprising SAP |
| Separate, optionally, in monomeric and dry form | Calcium in separate solution | Encapsulated in particles | - | e.g., phosphate-cross-linked chitosan particles |
| monomeric and dry form, e.g., spray coated on PVA pad with Ca | Calcium with SAP | Encapsulated in particles | | e.g., phosphate-cross-linked chitosan particles suspended in aqueous solution and PVA pad with SAP and encapsulated Ca |
| monomeric and dry form, e.g., spray coated on PVA pad | Encapsulated in Alginate matrix in shell of particles | Encapsulated in chitosan matrix coated with Alginate-Ca shell | | e.g., phosphate-cross-linked chitosan particles coated with alginate-Ca shell suspended in aqueous solution and SAP separately |
| monomeric and dry form, e.g., spray coated on PVA pad | Encapsulated in Alginate matrix in particles | Encapsulated in chitosan matrix in separate particles | | e.g., phosphate-cross-linked chitosan particles and alginate-Ca particles suspended in aqueous solution and SAP separately |
| monomeric and dry form, e.g., spray coated on PVA pad | Encapsulated in Alginate matrix | Encapsulated in chitosan matrix | | e.g., phosphate-cross-linked chitosan particles and alginate-Ca beads suspended in oily solution and SAP separately |

Optionally, a kit of the invention may comprise a dry composition comprising the particles and a liquid composition, preferably, water or an aqueous composition.

In one embodiment of the composition or kit of the invention, the particles are in a dry composition, optionally, in lyophilized form. Spray-drying is an alternative form of drying that has advantages if higher amounts of the compositions are to be produced. Dry compositions may be water-free. Drying, e.g., lyophilization with cryoprotectants, e.g, trehalose, sucrose and/or mannitol can help to maintain nanoparticle integrity during long-term storage.

Optionally, the SAP, if it is in a different component of a kit of the invention from the particles, may also be in a dry composition, e.g., as obtainable from the method of WO 2014/027012 A1. Dry storage of SAP has the advantage that, in accordance with the prior art, the SAP can be maintained in monomeric form. Thus, in one embodiment, the invention provides a kit comprising dry SAP as one component and dry particles as a second component. The particles may encapsulate calcium and/or phosphate, preferably both. The particles may e.g., be starch-based particles as described herein. They may be core-shell particles or homogenous particles.

A composition comprising the particles can also be a solution or dispersion, preferably, a water-based dispersion. The composition can be a toothpaste, a tooth gel, a mouthwash, a mouth spray, an oral care foam, a chewing gum, a toffee, a lozenge, a tablet, a powder, a putty, granules or a candy.

For example, as part of a kit, the self-assembling peptide may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy.

As part of a kit, the calcium ions may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy.

As part of a kit, the phosphate may be provided in a composition selected from the group consisting of a dry composition, a lyophilized composition, a water-free composition, a water-based solution, a toothpaste, a tooth gel, a mouthwash, a mouth spray, an oral care foam, a chewing gum, a toffee, a lozenge, a tablet, a powder, a putty, granules or a candy.

The kit of the invention may be provided in the form of a syringe, preferably, a multi-chamber syringe, which, can be advantageously used for targeted treatment, e.g., for targeting a tooth lesion such as a subsurface lesion. Separate components of the kit of the invention can also be packaged in a blister packaging, e.g., as described in EP 23 210 478.6.

Those forms intended to be chewed or sucked, e.g., chewing-gum, toffee, lozenge, candy, or also toothpaste, a tooth gel, a mouthwash, a mouth spray, an oral care foam are for use on treatment of caries lesions, while putty is typically used for bone treatment or pulp capping. Other forms can be used for both applications.

As stated, preferred compositions of the invention are solutions, e.g., in water. Alternative preferred compositions of the invention are dry, e.g., water-free powders that are suitable for being dissolved or, in the case of the non-soluble particles, resuspended in water.

The compositions may further comprise, optionally, e.g., water, oil, preservatives, pH adjusting agent, thickener, stabilizer and/or fragrance.

### Medical use

The composition or kit of the present invention may be used for research purposes, but preferably, it is for use in medicine.

Mainly, it is for use in inducing tooth remineralization or bone regeneration, preferably, in a subject in need thereof. The subject typically is a human subject. Advantageously, said remineralisation or regeneration is accelerated compared the absence of said calcium and phosphate ions.

In one embodiment, the kit of the invention is for use in inducing mineralization or remineralization in or on a tooth of a subject, preferably, for treating a tooth lesion or cavity. The tooth lesion may be a caries lesion. It may also be broken enamel or dentin, typically, broken enamel, or cavitated enamel or dentin. The kit of the invention may also be for use in filling pits and/or fissures, both in the presence or absence of lesions in said pits and fissures. A further use is for the treatment of tooth sensitivity, wherein dentinal tubuli may be occluded or blocked by a protective barrier made of the invention.

Preferably, the composition or kit of the invention are provided for use in treating at least one caries lesion, preferably, a subsurface caries lesion. It can also be for use in treating a cavitated caries lesion in combination with another filling agent, such as a glass-ionomer cement or a composite filling. In this case, it can contribute to reducing the incidence or preventing secondary caries. An alternative use is for treatment of a bone defect, with the aim of remineralisation.

Application of the composition of the invention or of components of the kit of the invention, in particular, of the particles, is optionally repeated, e.g., daily for at least 2 days, preferably, for a week.

In one embodiment, the composition or kit of the present invention is for use in pulp capping. Here, the material formed by after administering the composition or the components of the kit of the invention can protect the pulp from the often toxic effect of the dental sealant used above it, by placing a protective biocompatible layer of calcium phosphate over the dental pulp.

In said embodiment, optionally, the kit of the invention further comprises, separately from the other components, a dental sealant such as a glass-ionomer cement or a composite, or a dental sealant comprising components which are capable of polymerizing, such as acidic polymer selected from the group comprising an acrylate and methacrylate, ionomer, giomer, ormocer^{®} and any other suitable polymer and/or a monomeric form thereof.

Accordingly, the invention also provides a method of treating at least one caries lesion, e.g., a subsurface caries lesion or a cavitated caries lesion, comprising administering an effective amount of the composition of the invention or, after mixing or sequentially, the components of the kit of the invention to said at least one caries lesion.

The invention also provides a method of treating a bone defect, comprising administering an effective amount of the composition of the invention or, after mixing or sequentially, the components of the kit of the invention to said at least one bone lesion. The bone defect or bone lesion may e.g., be caused by a tumor or by trauma. The composition or kit may also be for use in augmentation or reconstructive treatment of the alveolar ridge, for filling of periodontal defect, or for filling of a defect after root resection, apicoectomy, cystectomy, for filling of an extraction socket to enhance preservation of the alveolar ridge, for elevation of the maxillary sinus floor, for filling a periodontal defect or a peri-implant defect. It can also be applied in orthopaedic indications such as joint implants (e.g. hip-implants) or spinal fusion.

Accordingly, the composition is a pharmaceutical composition, or the kit is a pharmaceutical kit.

In case one or more components of the kit or the composition is/are in dry form, it is dissolved in water or in an aqueous solution before administration. Water, if not mentioned otherwise, is deionized or distilled water at pH 7.

In summary, the kit and composition of the invention advantageously accelerate tooth remineralization or bone regeneration, e.g., compared to administration of either self-assembling peptide or calcium and/or phosphate alone. In one optional embodiment, release of cargo from the particles increases the pH of a subsurface lesion to be less acidic, optimally, to about pH 6.5-10. At less acidic or more basic pH, the balance between remineralisation and demineralisation is further shifted towards remineralisation. The invention also allows for a very targeted treatment, as preferably, the particles assemble in the lesions because of their characteristics, e.g., their surface charge, and, additionally, the cargo is released there in a targeted manner, such as because of pH effects or presence of enzymes.

The invention is further exemplified by the following embodiments, examples and figures, which are meant to illustrate, but not to limit the invention. All references cited herein are herewith fully incorporated.

In the context of the invention, "a" is intended to encompass the plural, i.e., "a tooth" also refers to a plurality of teeth, e.g., all teeth of a subject. In the context of core-shell particles, multi-core or multi-shell particles are possible, but particles with one core and one shell are preferred. About means +/- 10%.

### List of Embodiments

1. A composition or kit comprising
   a) a self-assembling peptide (SAP) comprising the amino acid sequence SEQ ID NO: 1,
   b) calcium,
   c) phosphate, and
   d) a polymer in the form of particles having a diameter of 10-900.000 nm encapsulating a cargo selected from at least one of the self-assembling peptide (SAP), the calcium ions and the phosphate ions.
2. The composition or kit of embodiment (E) 1, wherein the particles are nanoparticles having a diameter of 10-999 nm. optionally, 10-700 nm.
3. The composition or kit of embodiment (E) 2, wherein the nanoparticles have a diameter of 50-500 nm, optionally, 50-350 nm, e.g., 100-200 nm.
4. The composition or kit of E1, wherein the particles are microparticles having a diameter of 1.000-900.000 nm, optionally, of 2.500-7.500 nm, e.g., 3.000-5.000 nm.
5. The composition or kit of any of E1-E4, wherein the particles bear at least one cationic surface region for associating the particles with a subsurface caries lesion in a tooth in an oral cavity of a subject.
6. The composition or kit of any of E1-E5, wherein the particles have an overall net positive charge.
7. The composition or kit of any of E1-E6, wherein the particles have a positive zeta potential at pH 5.5 or less.
8. The composition or kit of any of E1-E7, wherein the particles have a positive zeta potential at pH 7.0.
9. The composition or kit of any of E1-E8, wherein the positive zeta potential is a zeta potential of +2 to +50 mV, e.g., +30 to +50 mV.
10. The composition or kit of any of E1-E9, wherein the positive zeta potential is a zeta potential of +2 to +30 mV, optionally, +2 to +20 mV.
11. The composition or kit of any of E1-E10, wherein the positive zeta potential is a zeta potential of +2 to +5 mV.
12. The composition or kit of any of E1-E11, wherein the particles are sensitive to a trigger leading to targeted release of their cargo, wherein the trigger is selected from the group consisting of a pH of 5.5 or lower, presence of amylase and/or body temperature.
13. The composition or kit of E12, wherein the particles are pH sensitive and release at least one of their cargo at a pH of 5.5 or lower, optionally, at least two of their cargos or all cargos.
14. The composition or kit of E12 or E13, wherein the presence of amylase triggers release of at least one cargo, and the particles comprise a carbohydrate sensitive to amylase, optionally, starch.
15. The composition or kit of any of E1-E14 wherein the polymer is selected from the group comprising a carbohydrate, a peptide, polyaspartic acid (PASP), poly(allylamine) hydropchloride (PAH), poly(N-isopropylacrylamide), poly(ethylene glycol)-block-poly(lactic acid), polyvinylalcohol (PVA), polyvinylacetate (PVAc), polyvinylpyrrolidone (PVP), polyethyleneimine (PEI), poly(L-lysine) (PLL), poly(L-arginine), poly(amidoamine) (PAA), poly (amino-co-ester) (PAE), poly(2-N,N-dimethylaminoethylmethacrylate) (PDMAEMA), poly(4-vinylpyridine) (P4VP), polyesters, poly(acrylic acid), poly(methacrylic acid), a polyalkylene glycol, a methyl vinyl ether/maleic anhydride copolymer, gelatin, and combinations thereof.
16. The composition or kit of E15, wherein the polymer is a biopolymer, such as a modified biopolymer or a non-modified biopolymer.
17. The composition or kit of claim of any of E15 or E16, wherein the at least one carbohydrate is selected from the group comprising alginate, chitosan, starch, pectin, cellulose, carboxymethylcellulose, hydroxypropylcellulose, hydroxyethylcellulose, dextrin and/or dextran.
18. The composition or kit E17, wherein the carbohydrate is alginate, optionally, in combination with chitosan.
19. The composition or kit of E17, wherein the carbohydrate is chitosan.
20. The composition or kit of E17, wherein the particle comprises both chitosan and alginate.
21. The composition or kit of E20, wherein the particle comprises a core comprising chitosan and a shell comprising alginate.
22. The composition or kit of E20, wherein the particle comprises a core comprising alginate and a shell comprising chitosan.
23. The composition or kit of E17, wherein the carbohydrate is starch.
24. The composition or kit of E23, wherein the starch comprises amylopectin.
25. The composition or kit of E23 or E24, wherein the starch comprises amylose.
26. The composition or kit of any of E23-E25, wherein the starch is a phosphate cross-linked starch,
   preferably, starch crosslinked with a crosslinker comprising sodium trimetaphosphate and wherein the particles further comprise calcium ions,
   wherein, optionally, the particles further comprise SAP.
27. The composition or kit of E17, wherein the carbohydrate is pectin.
28. The composition or kit of E17, wherein the carbohydrate is cellulose.
29. The composition or kit of E17, wherein the carbohydrate is carragenaan.
30. The composition or kit of E15, wherein the polymer is PVA.
31. The composition or kit of E12, wherein the trigger is body temperature and the polymer is selected from the group comprising poly(N-isopropylacrylamide), poly(ethylene glycol)-block-poly(lactic acid), and/or gelatin.
32. The composition or kit of any of E1-E31, wherein the particles comprise one polymer only, wherein SAP is not considered.
33. The composition or kit of any of E1-E31, wherein the particles comprise at least two polymers, wherein SAP is not considered, preferably two polymers.
34. The composition or kit of any of E1-E33, wherein the particles ae core-shell particles comprising a core, preferably one core, and at least one shell, preferably one shell.
35. The composition or kit of any of E1-E34, wherein release of cargo from the particles induces a pH change in a subsurface lesion such that the pH becomes less acidic or more basic, preferably, such that the pH in the subsurface lesion changes to pH 6-10, optionally, pH 6-8.
36. The composition or kit of any of E1-E35, comprising Ca(C₂H₃O₂)₂, CaCl₂, Ca(OH)₂, CaCO₃ and/or calcium lactate.
37. The composition or kit of E36, comprising Ca(C₂H₃O₂)₂.
38. The composition or kit of any of E36-E37, comprising CaCl₂
39. The composition or kit of any of E36-E38, comprising Ca(OH)₂.
40. The composition or kit of any of E36-E39, comprising CaCO₃.
41. The composition or kit of any of E1-E40, comprising sodium tripolyphosphate (TPP), sodium hexametaphosphate (SHMP), sodium phosphate (Na₃PO₄), potassium phosphate (K₃PO₄), disodium phosphate (Na₂HPO₄), monosodium phosphate (NaH₂PO₄), sodium pyrophosphate (Na₄P₂O₇), amorphous calcium phosphate (ACP), glycerol phosphate or covalently linked phosphate groups.
42. The composition or kit of E41, comprising sodium tripolyphosphate (TPP).
43. The composition or kit of any of E41-E42, comprising sodium phosphate.
44. The composition or kit of any of E41-E43, comprising disodium phosphate.
45. The composition or kit of any of E41-E44, comprising monosodium phosphate.
46. The composition or kit of any of E41-E45, comprising amorphous calcium phosphate.
47. The composition or kit of any of E1-E46 comprising both calcium ions and phosphate, wherein, optionally, the phosphate is a covalently linked phosphate group, e.g., covalently linked to the polymer.
48. The composition or kit of any of E47-E48, comprising calcium ions and phosphate ions each at least in the form of a salt having a solubility in water at 25°C and pH 7 of at least 7 g/100 mL.
49. The composition or kit of E47-E48, wherein both calcium ions and phosphate, e.g., phosphate ions are encapsulated in the particles.
50. The composition or kit of E49, wherein the particles are structured to release the majority of the calcium ions before the majority of phosphate ions.
51. The composition or kit of E50, wherein the particles comprise a shell comprising calcium ions and a core comprising phosphate, e.g., phosphate ions.
52. The composition or kit of E49, wherein the particles are structured to release the majority of the phosphate ions before the majority of calcium ions.
53. The composition or kit of E52, wherein the particles comprise a shell comprising phosphate, e.g., phosphate ions and a core comprising calcium ions.
54. The composition or kit of any of E1-53 further comprising fluoride ions.
55. The composition or kit of E54, wherein the fluoride ions are encapsulated in the particles.
56. The kit of any E54, wherein the fluoride ions are dissolved in an aqueous solution comprised in a compartment of the kit separate from the particles.
57. The composition or kit of any of E1-53 not comprising fluoride ions.
58. The composition or kit of any of E1-57, wherein the SAP is encapsulated in the particles.
59. The composition or kit of any of E1-58, wherein the SAP is in monomeric form.
60. The composition or kit of E58, wherein the SAP is in assembled form.
61. The composition or kit of any of E1-57, wherein the SAP is not encapsulated in the particles, but in one composition with the particles.
62. The composition or kit of E61, wherein the SAP is in monomeric form.
63. The composition or kit of E61, wherein the SAP is in assembled form.
64. The composition or kit of any of E1-63, wherein the self-assembling peptide comprises SEQ ID NO: 6 or an amino acid sequence having at least 80% identity thereto.
65. The composition or kit of E64, wherein the self-assembling peptide is P11-4 of SEQ ID NO: 6.
66. The composition or kit of any of E1-65, wherein the particles are nanoparticles having a diameter below 500 nm, wherein the particles further comprise an additional hydrophilic polymer (optionally, PEG), and/or a polyelectrolyte.
67. The composition or kit of any of E1-66, wherein the particles are nanoparticles having a diameter below 500 nm and wherein the composition comprising the nanoparticles comprises a surfactant.
68. The composition or kit of any of E1-67, wherein the particles are in a dry composition, optionally, in spray-dried or lyophilized form.
69. The composition of kit of claim 68, wherein the composition comprising the particles comprises a non-reducing sugar, optionally, trehalose, sucrose or mannitol, preferably, trehalose.
70. The composition or kit of any of E1-69, wherein the SAP are in a dry composition, optionally, in spray-dried or lyophilized form.
71. The composition of any of E1-E70.
72. The kit of any of E1-E70.
73. The kit of any of E68-E70, comprising the dry composition and a liquid composition, preferably, water or an aqueous composition.
74. The kit of E73, wherein the dry composition comprises both SAP and particles.
75. The kit of E73, wherein the particles and/or the SAP are associated with a solid support selected from the group comprising a sponge or pad or a film, wherein, optionally, the solid support is from a synthetic material such as PVA.
76. The kit of E75, wherein the solid support is a sponge.
77. The kit of any of E75 or E76, wherein the solid support is prepared by dip coating or spray drying, optionally, liquid bed spray drying.
78. The composition of any of E1-E71 that is a dental care product selected from the group comprising a mouth wash, a toothpaste, a tooth mousse, a solution for targeted application to a subsurface caries lesion, a chewy product or a film.
79. The composition of E78 that is a mouth wash.
80. The composition of E78 that is a paste or gel, optionally, a toothpaste.
81. The composition of E78 that is a tooth mousse.
82. The composition of E78 that is a solution for targeted application to a subsurface caries lesion.
83. The composition of E78 that is a chewy product selected from the group comprising a chewing gum, a toffee, a chewy candy or a chew toy.
84. The composition of E78 that is a film.
85. The composition of E84 wherein the film (also designated strip or biomembrane) comprises a polysaccharide, collagen, gelatine.
86. The composition of E84 or E85 wherein the film comprises a synthetic material selected from the group comprising Teflon, PVA and PLGA and a combination thereof.
87. The composition of any of E84-E86, wherein the film is prepared by dip coating or spray drying, optionally, liquid bed spray drying.
88. The composition of E78 that is a wax stick.
89. The composition or kit of any of E1-E88 for use in treating at least one caries lesion, preferably, a subsurface caries lesion.
90. A method of treating at least one caries lesion, comprising administering an effective amount of the composition of any of E1-E88 or, after mixing, the components of the kit of any of E1-E69 to at least one caries lesion,
   wherein, if the composition comprising the particles is in dry form, it is dissolved in an aqueous solution before administration.
91. A method of treating at least one caries lesion, comprising administering an effective amount of the components of the kit of any of E1-E77 to at least one caries lesion, wherein a component comprising SAP is administered first and a component comprising calcium and/or phosphate is administered afterwards,
   wherein, if any component is in dry form, it is dissolved in an aqueous solution before administration.
92. The composition or kit of any of E1-E88 for use in treating a bone defect.
93. A method of treating a bone defect, comprising administering an effective amount of the composition of any of E1-E88 or, after mixing, the components of the kit of any of E1-E69 to at least one bone lesion,
   wherein, if the composition comprising the particles is in dry form, it is dissolved in an aqueous solution before administration.
94. A method of treating a bone defect, comprising administering an effective amount of the components of the kit of any of E1-E77 to at least one bone lesion, wherein a component comprising SAP is administered first and a component comprising calcium and/or phosphate is administered afterwards,
   wherein, if any component is in dry form, it is dissolved in an aqueous solution before administration.
95. A pharmaceutical composition comprising the composition of any of E1-88 or a pharmaceutical kit of any of E1-E77.

### Examples

The present invention provides a novel targeted release polymeric system utilizing an encapsulation approach. The system aims to release calcium and phosphate ions in response to oral environmental triggers, such as pH variations, with the aim of maximizing their remineralization effect. Optionally, calcium and phosphate can be released sequentially. The methodology focuses on controlled release, biocompatibility, and *in situ* hydroxyapatite formation facilitated by self-assembling peptides (SAP) such as P11-4.

### 1) Preparation of particles

### 1A) Preparation of calcium-alginate nanoparticles

### Method 1

Alginate nanoparticles are obtained by inducing the gelation of a sodium alginate solution with calcium chloride, e.g., as disclosed in Rajaonarivony et al., 1993. Development of a New Drug Carried Made from Alginate. Journal f Pharmaceutical Sciences 82(9):P912-917.. Poly-L-lysine is then added to make a polyelectrolyte complex.

### STEPS

**1.** Preparation of Sodium Alginate Solution: Dissolve sodium alginate in distilled water to prepare a solution with a concentration of either 0.12% or 1% (w/v).
**2.** Addition of Calcium Chloride:
   - Prepare an 18 mM calcium chloride (CaCl₂) solution.
   - Add varying volumes (0.5-5 mL) of the calcium chloride solution to the alginate solution.
   - Stir the mixture magnetically to induce gelation and the formation of calcium alginate nanoparticles.
**3.** Addition of Poly-L-lysine:
   - Prepare a 0.05% (w/v) poly-L-lysine solution.
   - Add 2 mL of the poly-L-lysine solution to the calcium alginate nanoparticle suspension containing 0.06% alginate.
   - Stir the mixture to form a polyelectrolyte complex between the negatively charged alginate and positively charged poly-L-lysine.
   - pH Adjustment: Ensure the pH of the final suspension is around 6.8 to stabilize the nanoparticles.

### Method 2

As disclosed in Daemi et al., 2012 (Synthesis and characterization of calcium alginate nanoparticles, sodium homopolymannuronate salt and its calcium nanopartciles. Scietia Iranica 19(6):2023-2028), solutions of sodium alginate with different concentrations (0.03%, 0.06%, 0.12% w/v) are obtained by dissolving proper amounts of polymer in deionized water at room temperature. Then the required amounts of CaCl₂ are dissolved in deionized water to obtain clear solutions with precise concentration (18, 36 mM). After homogenization of sodium alginate solution by mechanical stirrer, the solution of calcium chloride is added to the system. After 1 h of the rotation, prepared nanoparticles are purified by ultracentrifugation for 20 min.

### 1B) Preparation of alginate nanospheres

Alginate nanospheres containing e.g. P11-4 can be prepared e.g., according to Yu et al., 2008. Fabrication of nanosheres and vesicles as drug carriers by self-assembly of alginate. The Journal of Physical Chemistry C 112(43): 16774-16778)
1. Weigh 0.10 g of sodium alginate and dissolve it in 5 mL of distilled water under continuous stirring.
2. Place the solution in a thermostatic water bath at 55°C for 30 minutes.
3. Prepare a 0.02 M solution of calcium hydroxide (Ca(OH)₂).
4. Add 1 mL of the Ca(OH)₂ solution dropwise to the sodium alginate solution, and stir the mixture for an additional 1 hour at 55°C.
5. Prepare a 0.03 M solution of sodium bicarbonate (NaHCO₅).
6. Add 3 mL of the NaHCO₃ solution dropwise to the system and stir for another 3 hours at 55°C.
7. Add API
8. Continue stirring the mixture at 55°C for 24 hours.
9. Rapidly cool the solution by placing it in a water bath at 20°C for 30 minutes.
10. Transfer the cooled mixture into a dialysis bag and dialyze against 500 mL of distilled water at 20°C for 24 hours.
11. Collect the precipitated drug-loaded nanospheres.

### 1C) Preparation of chitosan nanoparticles

Phosphate-containing chitosan nanoparticles optionally, also containing calcium, can be prepared, e.g., according to Jiang et al. 2024, Scientific Reports 14: 18754, but without herbicidal agent.

### For example, the preparation can comprise the following steps:

1. Dissolve chitosan in 1% acetic acid solution to prepare chitosan solution (pH ~4.7).
2. Prepare sodium tripolyphosphate (TPP) solution in water at specified concentrations.
3. Filter both solutions to remove aggregates.
4. Adjust the ionic strength of chitosan and TPP solutions by adding NaCl before mixing.
5. Use different mixing methods (e.g., dropwise addition, single-shot mixing, or dilution) to control particle size and polydispersity e.g. add TPP solution dropwise into chitosan solution under stirring for 30 minutes.
6. Optimize conditions such as NaCl concentration, chitosan/TPP ratio, and stirring speed to fine-tune the nanoparticle size
7. Separate nanoparticles by ultracentrifugation, measure encapsulation efficiency using HPLC, and characterize the particles using dynamic light scattering (DLS) and scanning electron microscopy (SEM).

The average chitosan/TPP particle size depends on many parameters such as ionic strength, mixing procedure, chitosan and TPP concentration, chitosan degree of deacetylation (DD), temperature and pH. In each of these cases, particle size depends on two factors: (1) their swelling properties, which depend on the pH, ionic strength and TPP:chitosan ratio and (2) their aggregation number, which is the number of aggregated chitosan chains in an average chitosan/TPP particle. Sawarie et al., 2017. Colloids and Surfaces B: Biointerfaces 157;110-117) teaches preparation of chitosan/tripolyphosphate nanoparticles with highly tunable size and low polydispersity.

### 1D) Preparation of core-shell nanoparticles comprising a calcium alginate shell and phosphate-chitosan core

1. Different methods can be used for preparation of core-shell particles, e.g., co-extrusion technology or sequential layering. Core Formation (Chitosan Phosphate): mix chitosan with phosphate.
   - Chitosan dissolves and shows good solubility in slightly acidic to neutral media due to the protonation of its amino groups, which helps it dissolve by forming an ionic complex with phosphate. A common pH range for the dissolution of chitosan in acid solution is around 4.5 to 6.0.
   - By maintaining the core at low pH, that is, under acidic conditions, calcium ions can be kept in a state whereby their interaction is mainly with the alginate shell and not with the phosphate in the core. The protonated amino groups of chitosan and the phosphate ions themselves should not form easily insoluble calcium phosphate in an acid environment. If the pH goes above 7.0, calcium ions may interact with phosphate to form precipitates of calcium phosphate, interfering with the action of the system.
2. Shell Formation (Alginate Shell with Calcium):
   - For calcium to crosslink the alginate, the pH should be neutral to slightly basic, typically around 6.0 to 7.5. At pH values below 6.0, there is weak, or incomplete, interaction from the calcium ions cross-linking with the alginate.
   - The alginate will crosslink with calcium.
   - The pH difference from the core (acidic) to the shell (neutral to slightly basic) enables the core of chitosan phosphate to remain intact without precipitating calcium phosphate, while still allowing the alginate shell to crosslink with calcium.
   - Sequential Layering: The alginate shell is formed by extruding the chitosan phosphate core into a calcium-free solution comprising alginate. The particles thus formed can then be exposed to a calcium solution. This way, the calcium will interact primarily with the alginate shell.
   - In a co-extrusion setup, the chitosan phosphate core is extruded through a nozzle and, at the same time, surrounded by the alginate solution (shell) as it exits.
   - The extrusion system can have a coaxial nozzle, where the inner nozzle delivers the chitosan phosphate core and the outer nozzle provides the alginate solution, the material at exit assumes a core-shell morphology.

For example, the particles can be prepared by any method as described in Bennacef et al., 2023, Mar. Drugs 21(4), 235.

### 1E) Preparation of core-shell nanoparticles comprising a calcium alginate core and phosphate-chitosan shell

Alginate - calcium nanoparticles coated with chitosan resulting in a positive charge and positive zeta potential that promotes localisation in subsurface caries lesions can be advantageously used.

For example, the co-extrusion technique or sequential layering can also be used to prepare particles having a different order of shell and core, with corresponding exchanged use of solutions.

The preparation generally entails the initial gelation of alginate using calcium ions, forming a pre-gel, which is subsequently complexed with chitosan to create nanoparticles. The process comprises:
- Alginate Pre-Gelation: Sodium alginate solution is prepared and its pH adjusted appropriately. Calcium chloride solution is then added dropwise under stirring to induce gelation, forming an alginate core.
- Chitosan Complexation: A chitosan solution, prepared in acetic acid and adjusted to the desired pH, is added to the alginate pre-gel. The mixture is stirred to facilitate polyelectrolyte complexation, resulting in the formation of chitosan-alginate nanoparticles.

Core-shell nanoparticles comprising a calcium alginate core and phosphate-chitosan shell can e.g. be prepared as described in Li et al., 2008. Int J Biomed Sci 4(3):221-228, without addition of nifedipine.

### 1F) Preparation of triple layer core-shell nanoparticles

For a triple layer approach, phosphate ions encapsulated in fat, preferably in liposomes, e.g., LIP-66 or in TPP-chitosan nanoparticles can in a multi-core approach be encapsulated in a calcium alginate matrix. A chitosan coating that may comprise additional phosphate can be added to provide nanoparticles having a positive zeta potential.

### 2) Sequential application of SAP and nanoparticles containing calcium and/or phosphate

- SAP, e.g, P11-4, in monomeric form (Curodont^{®} Repair Fluoride Plus, vVardis, Switzerland) is applied to a subsurface caries lesion according to the manufacturer's instructions, i.e., after removal of pellicle and etching.
- In a second step, nanoparticles, preferably, having a size of 10-500 nm and a positive zeta potential at pH 7 and/or at pH 5.5 or less (preferably, both) are applied to the subsurface caries lesion. They can be applied as a dispersion of particles. Alternatively, after the administration of SAP, the particles are applied in the form of an oral film.
- Application of nanoparticles is optionally repeated, e.g., daily for at least 2 days, preferably, for a week.

**2A)** In one experiment, the calcium and phosphate containing nanoparticles are starch based, e.g., starch-based calcium and phosphate containing nanoparticles as described in WO 2017070578 A1 or, alternatively, as described in WO 2019/191456 A1 are applied. They can be applied as a dispersion of particles. Alternatively, after the administration of SAP, the particles are applied in the form of an oral film such as CrystLCare^{™} Biorestorative, e.g., Fluoride-Free (or the fluoride-containing variant thereof).

**2B)** In one experiment, alginate based calcium and phosphate containing nanoparticles are used, e.g., calcium-crosslinked alginate nanoparticles, e.g., as prepared in example 1A, optionally, further encapsulating a phosphate. They can be applied, e.g., as a dispersion of particles. If they do not comprise phosphate, e.g., a phosphate buffer can be applied sequentially, or the dispersion of particles can be in a phosphate buffer.

**2C)** In one experiment, chitosan based calcium and phosphate containing nanoparticles are used, e.g., phosphate-crosslinked chitosan nanoparticles further encapsulating a calcium salt, e.g., as prepared in example 1C. They can be applied, e.g., as a dispersion of particles.

**2D)** In one experiment, alginate based calcium, but not phosphate containing nanoparticles, e.g., calcium-crosslinked alginate nanoparticles, e.g., as prepared in example 1A without phosphate are used in combination with chitosan based phosphate containing nanoparticles without calcium, e.g., phosphate-crosslinked chitosan nanoparticles, e.g., as prepared in example 1C without calcium. The mixture of particles can be applied, e.g., as a dispersion of particles.

**2E)** In one experiment, calcium and phosphate containing nanoparticles having a core-shell structure and comprising both chitosan and alginate are used, wherein the core is a phosphate-crosslinked chitosan core and the shell is a calcium-alginate shell, e.g., as prepared in example 1D. They can be applied, e.g., as a dispersion of particles.

**2F)** In another experiment, calcium and phosphate containing nanoparticles having a core-shell structure and comprising both chitosan and alginate are used, wherein the shell is a phosphate-crosslinked chitosan shell and the core is a calcium-alginate core, e.g., as prepared in example 1E. They can be applied, e.g., as a dispersion of particles.

**2G)** In another experiment, calcium and phosphate containing nanoparticles having a triple shell structure and comprising both chitosan and alginate are used, e.g., as prepared in example 1F. They can be applied, e.g., as a dispersion of particles

### Example 3: Dual pH responsive polymeric delivery system

Either calcium or phosphate ions, e.g., calcium ions, are encapsulated in a first (i.e. outer) shell of a polymer particle, which degrades in the acidic environment of a subsurface caries lesion to release said ions. The shell comprises a pH active agent, e.g., calcium hyroxide and/or calcium acetate. Thus, degradation of the shell also leads to a rise in pH. The following degradation of the core of the polymer particle - or optionally, a second (i.e., inner) polymeric shell - containing the other of phosphate or calcium ions, e.g., phosphate ions leads to release of said ions. The released phosphate ions can promote enamel remineralization by facilitating remineralisation of calcium and phosphate onto the surface of the enamel mediated by SAP which may e.g., be contained in the shell, and optionally also in the core of the particles. The increase in the pH further enhances remineralisation and hydroxyapatite formation, which is otherwise hampered by the acidic pH in the subsurface lesion.

## Claims

1. A composition or kit comprising
a) a self-assembling peptide (SAP) comprising the amino acid sequence SEQ ID NO: 1,
b) calcium,
c) phosphate, and
d) a polymer in the form of particles having a diameter of 10-900.000 nm encapsulating a cargo selected from at least one of the self-assembling peptide (SAP), the calcium and the phosphate.

2. The composition or kit of claim 1, wherein the particles are nanoparticles have a diameter of 10-500 nm.

3. The composition or kit of any of the preceding claims, wherein the particles have a positive zeta potential at pH 5.5 or pH 7, preferably, at pH 5.5-7, optionally, a zeta potential of +2 to +50 mV.

4. The composition or kit of any of the preceding claims, wherein the particles are sensitive to a trigger leading to targeted release of their cargo, wherein the trigger is selected from the group consisting of pH, presence of amylase or lipase, and/or presence of water.

5. The composition or kit of any of the preceding claims, wherein the particles are pH sensitive and release at least one of their cargo at a pH of 5.5 or lower.

6. The composition or kit of any of the preceding claims, wherein the polymer is selected from the group consisting of a carbohydrate, a peptide, polyaspartic acid (PASP), poly(allylamine) hydropchloride (PAH), poly(N-isopropylacrylamide), poly(ethylene glycol)-block-poly(lactic acid), polyvinylalcohol (PVA), polyvinylacetate (PVAc), polyvinylpyrrolidone (PVP), polyethyleneimine (PEI), poly(L-lysine) (PLL), poly(L-arginine), poly(amidoamine) (PAA), poly (amino-co-ester) (PAE), poly(2-N,N-dimethylaminoethylmethacrylate) (PDMAEMA), poly(4-vinylpyridine) (P4VP), polyesters, poly(acrylic acid), poly(methacrylic acid), a polyalkylene glycol, a methyl vinyl ether/maleic anhydride copolymer, gelatin, and combinations thereof.

7. The composition or kit of claim 6, wherein the polymer is a carbohydrate selected from the group comprising alginate, chitosan, starch, pectin, cellulose, carboxymethylcellulose, dextrin and/or dextran, wherein, optionally, the carbohydrate comprises alginate, e.g., alginate in combination with chitosan.

8. The composition or kit of any of the preceding claims, wherein release of cargo from the particles induces a pH change in a subsurface lesion such that the pH becomes less acidic, preferably, such that the pH in the subsurface lesion changes to pH 6-10, optionally, pH 6-8.

9. The composition or kit of any of the preceding claims comprising a composition comprising both calcium and phosphate, wherein, preferably, both calcium and phosphate are encapsulated in the particles, wherein, optionally, the particles are structured to release the majority of the calcium ions before the majority of phosphate ions.

10. The composition or kit of any of the preceding claims, wherein the self-assembling peptide comprises SEQ ID NO: 6 or an amino acid sequence having at least 80% identity thereto,
optionally, wherein the self-assembling peptide is P11-4 consisting of the amino acid sequence of SEQI ID NO: 6.

11. The composition or kit of any of the preceding claims, wherein the SAP is encapsulated in the particles, optionally, wherein the SAP is in assembled form.

12. The composition or kit of any of claims 1-10, wherein the SAP is not encapsulated in the particles, but in one composition mixed with the particles, optionally, wherein the SAP is in monomeric form.

13. The kit of any of claims 1-10, wherein the SAP is separate from the particles, optionally, wherein the SAP is in monomeric form.

14. The composition of any of claims 1-12 or the kit of any of the preceding claims, wherein the particles are in dry form, optionally, in lyophilized or spray-dried form.

15. The composition of any of claims 1-12 or 14 or the kit of any of the preceding claims for use in treating at least one caries lesion or bone lesion, preferably, a subsurface caries lesion.

16. A method of treating at least one caries lesion or bone lesion, comprising administering an effective amount of the composition of any of the claims 1-12 or 14-15 or the components of the kit of any of the preceding claims to the at least lesion,
wherein, if the composition or any part of the kit is in dry form, it is dissolved in an aqueous solution or water before administration.
